# EUROPEAN PATENT SPECIFICATION

(11) **EP 4 561 456 B1**
(45) Date of publication and mention of the grant of the patent: **22.04.2026**
(21) Application number: 23753814.5
(22) Date of filing: 27.07.2023
(51) Int. Cl.: A61B 17/04, A61B 17/06, A61B 90/00, A61B 17/00

(54) **TISSUE CLOSURE DEVICE**
GEWEBEVERSCHLUSSVORRICHTUNG
DISPOSITIF DE FERMETURE DE TISSU

(30) Priority: 28.07.2022 CN 202210900219
(43) Date of publication of application: 04.06.2025
(73) Proprietor: Cilag GmbH International, 6300 Zug (CH)
(72) Inventor: YANG, Mengli, Shanghai (CN); DING, Sheng, Shanghai (CN); HE, Sijin, Shanghai (CN); LIU, Qinglong, Shanghai (CN)
(74) Representative: van Wanrooij, Eva
(86) International application number: PCT/EP2023/070859
(87) International publication number: WO 2024/023227

(56) References cited:
- US-A1- 2004 068 273
- US-A1- 2008 033 459
- US-A1- 2011 270 282

## Description

### TECHNICAL FIELD

The present invention relates to the technical field of tissue closure, and relates specifically to a tissue closure device.

### BACKGROUND OF THE INVENTION

During an operation, surgeons usually need to use tissue closure instruments to suture a patient's wound to be sutured, such as an incision of the abdominal cavity.

When suturing is carried out, existing tissue closure instruments need to use a puncture needle to pass through tissue from the exterior of the skin into the abdominal cavity. During actual operation, due to differences between individuals, when the puncture needle pierces into the abdominal cavity from the outside to the inside, the needle tip of the puncture needle may cause damage to the surrounding organs in the abdominal cavity. Therefore, there is typically a need for experienced surgeons to carry out puncturing.

In addition, when using existing tissue closure instruments, surgeons need to insert the puncture needle to a puncture position from one side, then draw out the puncture needle, and then insert the puncture needle to the puncture position from the other side, so as to arrange a suture thread in place to complete suturing. However, such means of puncturing may cause the deviation of the puncture position, which may affect the effective healing of the wound.

US 2008/0033459 A1 discloses a device for closing wounds created to access operative sites in a patient's body. The device comprises an elongated body with its features in profile to allow use through conduits such as trocars which can be removed over the device. The device has diametrically opposed pivoting extensions which provide a target for flexible needles to attach to suture and provide protection for vital structures in the body. Once the pivoting extensions are past the innermost layer adjacent to the wound the flexible needles are advanced through the tissue to obtain an amount of tissue to allow for a closure. The device leaves a suture behind which can then be tied to close the tissue defect.

US 2011/0270282 A1 discloses a surgical suture positioning system for closing an opening inside an intracorporeal tissue wall, comprising an elongated shaft, which has a distal shaft region, on which a means is provided, which is made at least partially of an elastomer material and which can be reversibly transferred from a position that is flush with the shaft into a position radially protruding over the shaft, and a needle guide unit, which is provided on the shaft and in which at least one needle arranged movably along the shaft is guided, which needle can be reversibly transferred from a retracted position into an advanced distal position, in which the needle penetrates the means located in the position radially protruding over the shaft, wherein a suture carried along by the means remains in the means by retracting the needle into the retracted position. The means has limbs that can be radially expanded away from the shaft and are operatively connected to an element such that the limbs can be reversibly transferred from the position that is flush with the shaft into the position radially protruding over the shaft.

US 2004/0068273 A1 discloses an incision closing apparatus for simultaneous insertion of suture needles through adjacent sides of an incision in a fascia layer within a patient. An insertion end is sized for insertion through the incision and a shield is retractably extended from the insertion end positioned interior of the fascia layer. A plurality of elongated spokes having needles thereon are extended from openings within the insertion end for ejection of needles having attached suture filaments through adjacent sides of the fascia layer incision. Each needle is imbedded into the extended shield to retain each needle from piercing an internal organ. Upon retraction of the shield into the insertion end, with each needle imbedded in the shield, the insertion end is removed from the incision, leaving suture filaments inserted through adjacent sides of the fascia layer incision for closure of the incision within the patient.

### SUMMARY

The objective of the present invention is to avoid the risk of damaging other organs around tissue when a suture needle is used to puncture tissue and to achieve consistency of puncture positions, thereby improving the safety of surgical operations.

In order to achieve the objective described above, the present invention provides a tissue closure device as recited in claim 1. Optional features are recited in the dependent claims.

In the tissue closure device, the needle manipulator can drive the plurality of suture needles to simultaneously move, so as to simultaneously extend obliquely from the needle outlet channels and pass through the tissue to move to the puncture position. In this way, the needle manipulator can drive the plurality of suture needles to the puncture position at one time, so that the consistency and symmetry of the puncture position can be improved, and the deviation of the puncture position can be avoided, so as to avoid the risk of damaging other organs around the tissue when the suture needles are punctured against the tissue, thereby improving the safety of surgical suturing.

Also disclosed is a tissue closing method which, although not explicitly claimed can be carried out by a tissue closure device according to the present invention. The tissue closing method comprises: arranging a suture thread on the exterior of a tube body of a tissue closure device, and positioning at least two ends of the suture thread at two needle outlet channels of the tube body, respectively; unfolding, by means of a driver on the tube body, stabilizers in an inner cavity of tissue to be sutured, and abutting the stabilizers against an inner cavity wall of the tissue; driving, by means of a needle manipulator, a plurality of suture needles in the tube body to simultaneously extend obliquely from corresponding needle outlet channels, such that two ends of the suture thread are drawn to pass through the tissue to be sutured and suture thread fixing portions of the stabilizers and then move to a puncture position; driving, by means of the needle manipulator, the plurality of suture needles back into the tube body, and retaining the two ends of the suture thread on the corresponding suture thread fixing portions, respectively; and folding the stabilizers by means of the driver on the tube body to draw out the two ends of the suture thread from a wound to be sutured.

In this tissue closing method, since the needle manipulator can drive the plurality of suture needles to simultaneously move, so as to simultaneously extend obliquely from the needle outlet channels and pass through the tissue to then move to the puncture position, as such, the needle manipulator can drive in one step the plurality of suture needles to the puncture position, so that the consistency and symmetry of the puncture position can be improved, and the deviation of the puncture position can be avoided, so as to avoid the risk of damaging other organs around tissue when the suture needles puncture the tissue, thereby improving the safety of surgical suturing.

At least two ends of the suture thread may be arranged in wiring notches on the tube body that are in communication with needle outlet channels, so that the at least two ends of the suture thread entering the wiring notches are located in thread-hanging ports of two suture needles, respectively.

At least two ends of the suture thread may be arranged in clamping slits of the wiring notches, so that the at least two ends of the suture thread clamped in the clamping slits are located in two of the thread-hanging ports, respectively.

After the plurality of suture needles respectively pass through the suture thread fixing portions of the stabilizers, the plurality of suture needles may drive the needle tip protective structure of the tissue closure device from a retracted position to a shielding position, and during the process of driving from the retracted position to the shielding position and in the shielding position, the needle tip protective structure shields needle tips of the plurality of suture needles.

When the needle manipulator drives the plurality of suture needles to be in the puncture position, the needle manipulator may block the driver from folding the stabilizers.

### BRIEF DESCRIPTION OF DRAWINGS

To better understand the above and other objectives, features, advantages and functions of the present invention, reference may be made to preferred embodiments shown in the drawings. The same or similar reference signs in the drawings refer to the same or similar components. It should be understood by those skilled in the art that the drawings are intended to schematically illustrate the preferred embodiments of the present invention and have no limitation on the scope of the present invention, and components are not drawn to scale.
FIG. 1 is a three-dimensional structural schematic diagram of one state of the tissue closure device provided in a specific embodiment of the present invention, wherein the needle manipulator is in the needle triggering position, and the stabilizers are in the unfolded position.
FIG. 2 is a three-dimensional structural schematic diagram of another state of the tissue closure device of FIG. 1, wherein the needle manipulator is in the needle initial position, and the stabilizers are in the folded position.
FIG. 3 is a three-dimensional structural view of the tissue closure device of FIG. 1 when the tube body is removed.
FIG. 4 is a three-dimensional structural view of the tissue closure device of FIG. 2 when the tube body is removed.
FIG. 5 is a three-dimensional structural view of the tissue closure device of FIG. 1 when one half tube portion of the tube body is removed.
FIG. 6 is a three-dimensional structural schematic diagram of FIG. 5 at another viewing angle.
FIG. 7 is a three-dimensional structural view of the tissue closure device of FIG. 2 when one half tube portion of the tube body is removed.
FIG. 8 is a structural schematic diagram of a cross-sectional view of the tissue closure device of FIG. 1 at one position.
FIG. 9 is a three-dimensional structural schematic diagram of the needle manipulator of the tissue closure device of FIG. 1 at one viewing angle.
FIG. 10 is a three-dimensional structural schematic diagram of the needle manipulator of FIG. 9 at another viewing angle.
FIG. 11 is a three-dimensional structural schematic diagram of the driver of the tissue closure device of FIG. 1 at one viewing angle.
FIG. 12 is a three-dimensional structural schematic diagram of the driver of FIG. 11 at another viewing angle.
FIG. 13 is a three-dimensional structural schematic diagram of one half tube portion of the tube body of the tissue closure device of FIG. 1 at one viewing angle.
FIG. 14 is a three-dimensional structural schematic diagram of two half tube portions of the tube body of the tissue closure device of FIG. 1 at one viewing angle.
FIG. 15 is an exploded state view of the cooperation of the needle manipulator and the suture needles of the tissue closure device of FIG. 1.
FIG. 16 is a partial structural schematic diagram of the cooperation of the needle manipulator, suture needles, driver, and driving rod together of the tissue closure device of FIG. 1.
FIG. 17 is a schematic diagram of the first needle tip protective structure of the tissue closure device provided in a specific embodiment of the present invention, wherein the protective plate is in the shielding position.
FIG. 18 is a schematic side structural diagram of FIG. 17.
FIG. 19 is a three-dimensional diagram of the protective plate of the needle tip protective structure in FIG. 17 in the retracted position.
FIG. 20 is a schematic side structural diagram of FIG. 19.
FIG. 21 is a schematic diagram of the second needle tip protective structure of the tissue closure device provided in a specific embodiment of the present invention, wherein the protective plate is in the shielding position.
FIG. 22 is a schematic diagram of a third needle tip protective structure of a tissue closure device provided in a specific embodiment of the invention, wherein the protective plate is in the shielding position.
FIG. 23 is a three-dimensional diagram of the protective plate in FIG. 22, wherein the protective plate is in a natural state in the retracted position.
FIG. 24 is a three-dimensional diagram of the stabilizer in FIG. 22.
FIG. 25 is a three-dimensional diagram of another protective plate, wherein the protective plate is in a natural state in the retracted position.
FIG. 26 is a three-dimensional diagram of another protective plate, wherein the protective plate is in a natural state in the retracted position.
FIG. 27 is a three-dimensional diagram of another protective plate, wherein the protective plate is in a natural state in the retracted position.
FIG. 28 is a three-dimensional diagram of another protective plate, wherein the protective plate is in a natural state in the retracted position.
FIG. 29 is a three-dimensional diagram of another protective plate, wherein the protective plate is in a natural state in the retracted position.
FIG. 30 is a partial structural diagram of the tube body of the tissue closure device provided in a specific embodiment of the present invention, which shows a wiring notch.
FIG. 31 is a partial structural diagram of the tube body of FIG. 30 at another viewing angle, showing a wiring notch.
FIG. 32 is a schematic side structural diagram of the tube body in FIG. 30.
FIG. 33 is a structural schematic diagram showing an internal suture needle.
FIG. 34 is a partial structural diagram of the tube body of the tissue closure device provided in a specific embodiment of the present invention, which shows another wiring notch.
FIG. 35 is a partial structural diagram of the tube body of the tissue closure device provided in a specific embodiment of the present invention, which shows the wiring structure.

### Description of reference signs

1: tube body, 2: needle outlet channel, 3: suture needle, 4: stabilizer, 5: needle manipulator, 6: driver, 7: needle tip protective structure, 8: manual operation portion, 9: guide slot, 10: guide bump, 11: needle insertion hole, 12: radial hole, 13: locking pin, 14: suture needle channel, 15: rectilinear recess section, 16: curvilinear recess section, 17: half tube section, 18: suture needle channel recess, 19: driving rod, 20: central hole, 21: radial channel, 22: locking shaft, 23: driving rod channel recess, 24: driving rod accommodating channel, 25: engagement opening, 26: driving opening, 27: fastener, 28: driving portion, 29: suspension arm, 30: guide recess, 31: wiring notch, 32: inlet section, 33: clamping slit, 34: thread-withdrawing notch, 35: elastic block, 36: wiring slot, 37: protective protrusion, 38: grid-like protrusion, 39: suture thread fixing portion, 40: thread-hanging port, 41: axial channel, 42: protective plate, 43: spring, 44: clamping slot, 45: clamping edge, 46: protective surface, 47: accommodating cavity, and 48: thread-pressing block.

### DETAILED DESCRIPTION

In the detailed description of the embodiments below, reference is made to the drawings which form a part of the description. The drawings illustrate, by way of example, specific embodiments in which the present invention is implemented. The illustrated embodiments are not intended to be exhaustive according to all of the embodiments of the present invention. It can be understood that other embodiments may be used, and structural or logical changes may be made without departing from the scope of the present invention as defined by the claims. With regard to the drawings, directional terms such as "lower", "upper", "left", and "right" are used with reference to the orientation of the drawings described. Since the assemblies of the embodiments of the present invention can be implemented in various orientations, these directional terms are used for illustrative purposes, rather than for restrictive purposes. Therefore, the following detailed description is not intended to be limiting, and the scope of the present invention is defined by the appended claims.

In addition, in the present invention, a "proximal end" mentioned below is an end of the tissue closure device facing an operator when in use, and a "distal end" is an end of the tissue closure device away from the operation when in use.

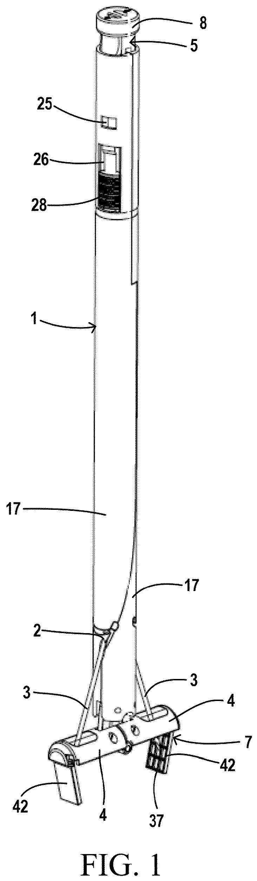

In the first aspect, with reference to FIGS. 1-4, the tissue closure device provided in a specific embodiment of the present invention includes a tube body 1, a plurality of suture needles 3, and a needle manipulator 5, wherein a plurality of needle outlet channels 2 are formed in the tube body 1, the plurality of suture needles 3 are located in the tube body 1, and the needle manipulator 5 is disposed on the tube body 1 and movable between a needle initial position and a needle triggering position; and wherein the needle manipulator 5 can drive the plurality of suture needles 3 to simultaneously move so as to simultaneously extend obliquely from respective corresponding needle outlet channels 2 to a puncture position; and the needle manipulator 5 allows the plurality of suture needles 3 to simultaneously move so as to simultaneously retract into the respective corresponding needle outlet channels 2.

In the tissue closure device, since the needle manipulator 5 can drive the plurality of suture needles 3 to simultaneously move, so as to simultaneously extend obliquely from the needle outlet channels 2 and pass through tissue to then move to the puncture position. In this way, the needle manipulator 5 can drive in one step the plurality of suture needles 3 to the puncture position, so that the consistency and symmetry of the puncture position can be improved, and the deviation of the puncture position can be avoided, thereby improving the safety of surgical suturing.

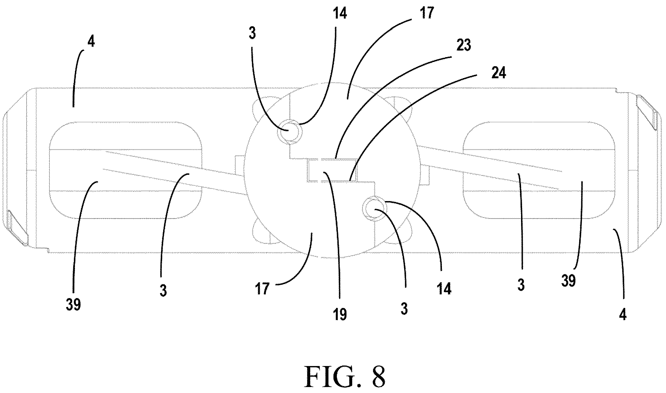

In addition, in the tissue closure device, in some embodiments, the plurality of suture needles can be located directly in an inner cavity of the tube body 1, and the needle manipulator 5 can drive the plurality of suture needles 3 to simultaneously extend obliquely from the needle outlet channels 2 by means of other possible structures. Alternatively, in other embodiments, with reference to FIG. 8, a plurality of independent suture needle channels 14 are provided in the tube body 1, each suture needle 3 is movably located in a respective suture needle channel 14, and each suture needle channel 14 includes a curvilinear channel section. In this way, the suture needles 3 may be bent along the curvilinear channel sections, so that the plurality of suture needles 3 can simultaneously extend obliquely from the respective corresponding needle outlet channels 2 to the puncture position. As such, by means of the suture needle channels 14, the suture needles 3 may, according to a required movement path, further stably and reliably extend from the needle outlet channels 2 or retract into the tube body 1, and at the same time, the curvilinear channel sections may also further ensure that the suture needles 3 can extend, according to a required tilt angle, from the needle outlet channels 2 to a predetermined puncture position.

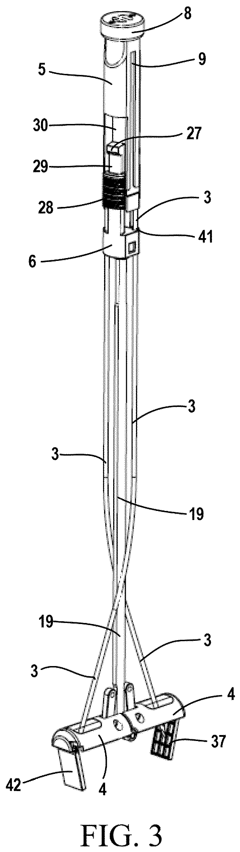

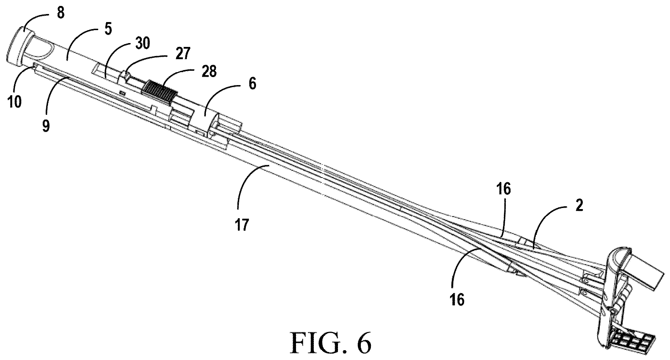

In addition, in the tissue closure device, the suture needles 3 have flexibility and bendability, so that under the limitation of the shape of the suture needle channels 14, the plurality of suture needles 3 can reach the puncture position under the driving of the needle manipulator 5. In some embodiments, with reference to FIGs. 3, 6, and 8, when the plurality of suture needles 3 are spatially cross-arranged, connection lines between the distal ends of the plurality of suture needles 3 are generally on a lateral (radial) center line of the tissue closure device, which can further accurately control the consistency and symmetry of the puncture position of the needle tips of the suture needles.

In addition, the curvilinear channel sections may have any shape that allows for the movement of the suture needles 3 and may be located at any desired positions of the suture needle channels 14. For example, the suture needle channels 14 may each include a plurality of curvilinear channel sections located at desired positions. For example, the suture needle channels 14 may be formed by sequentially connecting a plurality of identical or different curved sections.

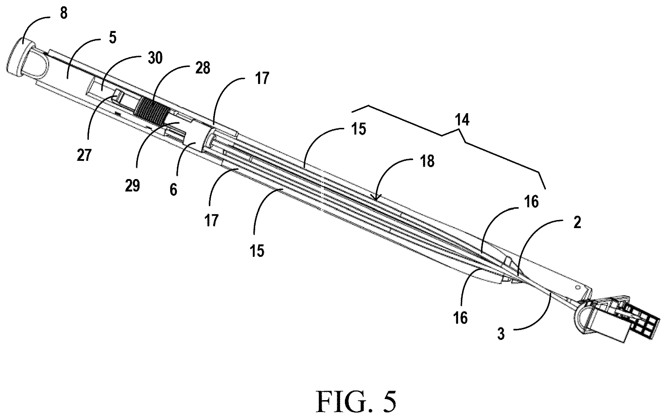

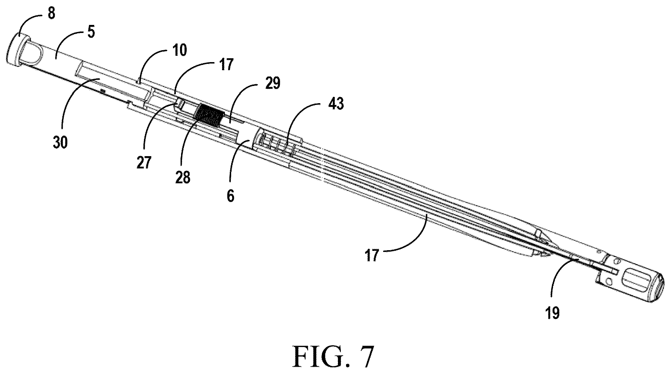

In addition, in the tissue closure device, in order to improve the smoothness of the movement of the suture needles 3 and to allow operation of the suture needles 3 to be easier for an operator, in some embodiments, with reference to FIGs. 5-7, each suture needle channel 14 includes a rectilinear channel section and a curvilinear channel section, so that the rectilinear channel sections may facilitate the suture needles 3 to move in the suture needle channel 14, while the curvilinear channel section may enable the plurality of suture needles to extend obliquely from the respective corresponding needle outlet channels 2 to the puncture position. For example, in some embodiments, the spatially cross-arranged curvilinear channel sections may allow the plurality of suture needles to extend obliquely in a spatially crossed manner from the respective corresponding needle outlet channels 2 to the puncture position.

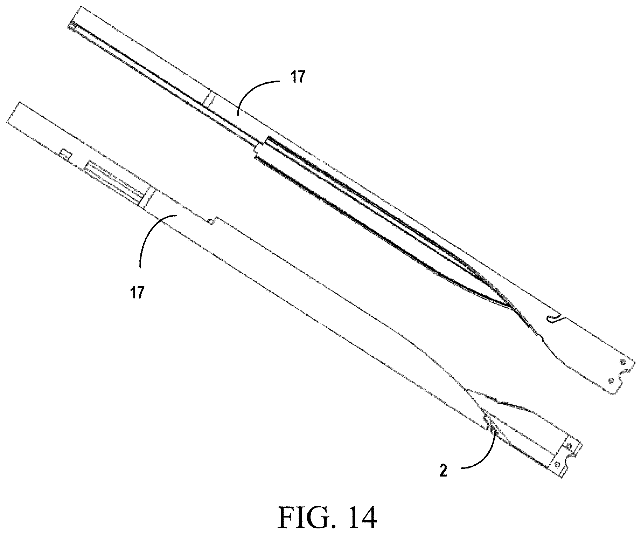

In addition, in the tissue closure device, in some embodiments, the rectilinear channel sections and the curvilinear channel sections may be located on the same side of the tube body 1. For example, on a cross section of the tube body 1, the rectilinear channel sections are arranged close to an edge of the tube body 1 while the curvilinear channel sections are arranged to extend at the edge of the same side. Alternatively, in other embodiments, with reference to FIG. 14, in the radial direction of the tube body 1, the rectilinear channel sections are located on one side of the tube body 1, the curvilinear channel sections extend from one side of the tube body 1 to the other side, and one end of each curvilinear channel section is used as a needle outlet channel 2 or is in communication with a needle outlet channel 2. As such, the interior of the tube body 1 can be fully utilized to rationally arrange the suture needle channels 14, so that the needle manipulator 5 more easily drives the plurality of suture needles 3, e.g., two suture needles 3 as shown, to move. For example, in FIGs. 3-8, two suture needle channels 14 are spatially cross-arranged. For example, two suture needle channels 14 are arranged symmetrically relative to the center of the tube body 1.

In addition, in the tissue closure device, the tube body 1 may have a variety of types. For example, in some embodiments, the tube body 1 may be an injection-molded integral piece. Alternatively, in other embodiments, the tube body 1 may be formed by a plurality of members being fitted together, so that it is easier to form suture needle channels 14 in which suture needles are arranged. For example, in some embodiments, the tube body 1 may include a plurality of tube sections, at least some of which have corresponding axial channels. The plurality of tube sections may be sequentially connected along an axial direction to form one tube body, while a plurality of axial channels are in communication with one another to form the suture needle channels 14. In some other embodiments, with reference to FIGs. 1, 13, and 14, the plurality of members includes two half tube portions 17, that is, the tube body 1 includes two half tube portions 17 that can be fitted together. A desired number of half-suture-needle channel recesses 18, e.g., one half-suture-needle channel recess 18 or a plurality of mutually independent half-suture-needle channel recesses 18, are formed on each half tube portion 17. When the two half tube portions 17 are combined to form the tube body 1, for example, the plurality of half-suture-needle channel recesses 18 on one half tube portion 17 and the plurality of half-suture-needle channel recesses 18 on the other half tube portion 17 are separately fitted to form a plurality of suture needle channels 14. The two half tube portions 17 may be bonded, clamped, thermally fused or connected together by other means. By means of the described two half tube portions 17, it may be easier to form suture needle channels 14 of desired shape. Of course, in other embodiments, the plurality of members may be three or four tubes, that is, the cross section of the tube body 1 is divided into three or four parts.

For example, in some embodiments, when assembling, the suture needles 3 and the needle manipulator 5 may be first placed on one half tube portion 17, and then the other half tube portion 17 is fitted and connected on the one half tube portion 17. In this way, the suture needles 3 are stably and reliably accommodated in the suture needle channels 14 of the tube body and can stably and reliably move along the suture needle channels, and the needle manipulator 5 is conveniently located in the tube body, thereby improving the convenience of assembling the tissue closure device. In addition, the suture needles may stably move by means of the guidance of the suture needle channels 14 for the suture needles 3, avoiding deviation of the puncture position and improving the healing effect of a sutured wound.

In addition, in some embodiments, each suture needle channel recess 18 includes a rectilinear recess section 15 located on one side in a radial direction of the tube body 1 and a curvilinear recess section 16 extending from one side to the other side. As such, the rectilinear recess sections 15 may be fitted to form a rectilinear channel section, and the curvilinear recess sections 16 may be fitted to form a curvilinear channel section. In some embodiments, with reference to FIGs. 5-7, 13 and 14, the fitting surface of each half tube portion 17 includes a curved surface section, and the curvilinear recess sections 16 are formed on the curved surface sections and extend along the extending direction of the curved surface. In this way, the curvilinear recess sections 16 may extend from one side to the other side, and when the two half tube portion recesses 17 are fitted, the two curvilinear recess sections 16 are fitted to form a curvilinear channel section. For example, suture needles may be placed in the half-suture-needle channel recess 18 of one half tube portion 17, and then the other half tube portion 17 is fitted on the one half tube portion 17, so that the suture needles can be arranged in suture needle channels 14.

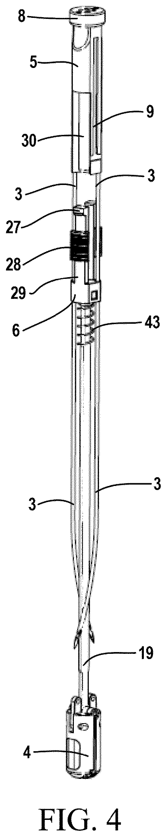

In addition, in some embodiments, with reference to FIGs. 3 and 4, the tissue closure device includes stabilizers 4 and a driver 6, wherein the stabilizers 4 are disposed on the tube body 1 and movable between an unfolded position and a folded position; wherein the driver 6 is disposed in the tube body 1 and movable between a stabilizer initial position and a stabilizer triggering position; and wherein the driver 6 can drive the stabilizers 4 to move, in the stabilizer initial position, the stabilizers 4 are in the folded position, and in the stabilizer triggering position, the stabilizers 4 are in the unfolded position. The number of stabilizers 4 may be one or more, for example, two. In this way, the distal end of the tube body 1 can be inserted into a composition to be sutured, and then the driver 6 is moved from the stabilizer initial position to the stabilizer triggering position so that the stabilizers 4 move from the folded position to the unfolded position. Thus, when pulling the tube body 1 outward, the stabilizers 4 can stably and reliably abut against the inner wall of the tissue to be sutured.

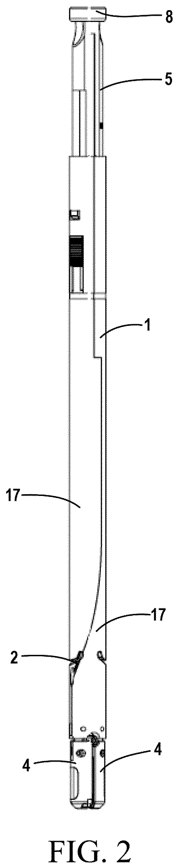

In addition, in some embodiments, the tissue closure device may not be provided with a needle tip protective structure 7. Alternatively, in some embodiments, in order to improve safety, when moving the suture needles to the puncture position, in order to prevent the needle tips of the suture needles from causing damage to surrounding organs, with reference to FIGs. 1 and 2, the tissue closure device includes a needle tip protective structure 7 having a retracted position and a shielding position, wherein the suture needles 3 can drive the needle tip protective structure 7 from the retracted position to the shielding position.; and when driving from the retracted position to the shielding position, and in the shielding position, the needle tip protective structure 7 shields the needle tips of the suture needles 3. As such, during the puncturing process of the suture needles 3 and at the shielding position, the needle tip protective structure 7 can always shield the needle tips of the suture needles 3, thereby avoiding exposure of the needle tips, and effectively avoiding the risk of the needle tips possibly causing damage to surrounding organs.

In the tissue closure device, the needle tip protective structure 7 may be of a variety of types. For example, in one type, the needle tip protective structure 7 may include a body and a protective cap, the protective cap may be connected to the body by means of a flexible connecting strip, and the protective cap may be located on the movement path of a suture needle. In this way, during the puncturing process of the suture needle 3 and at the shielding position, the protective cap may be sleeved on the needle tip to avoid exposure of the needle tip, and the flexible connecting strip may always connect the protective cap to the body so as to prevent the protective cap from falling off.

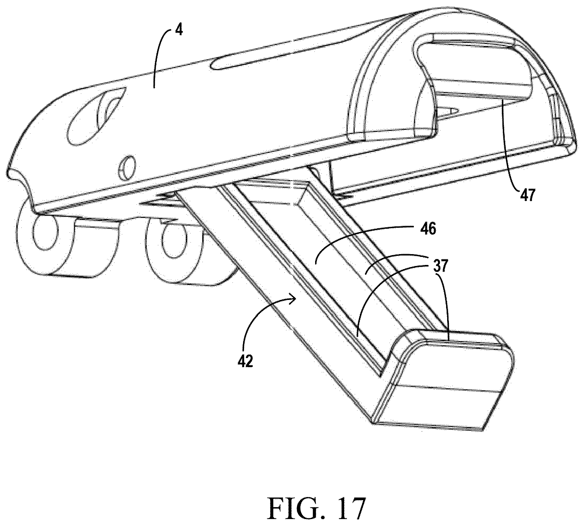

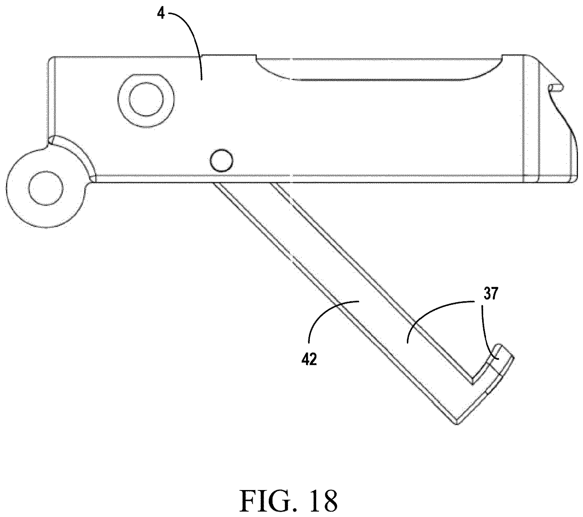

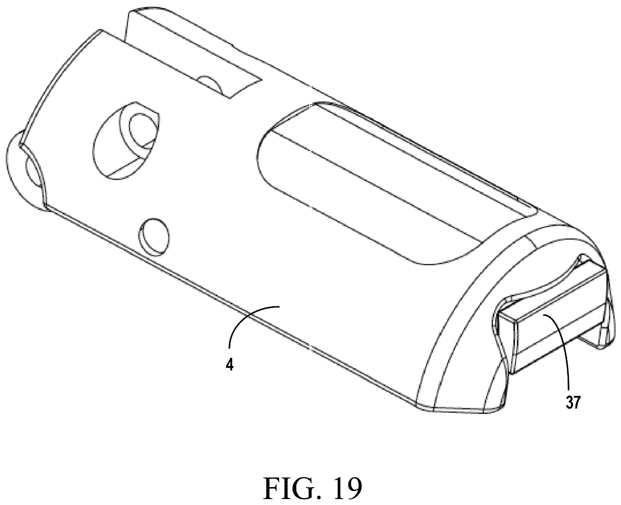

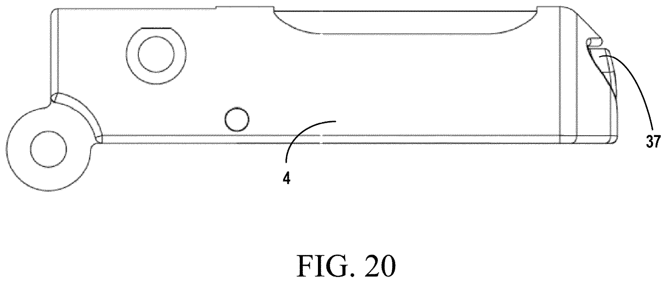

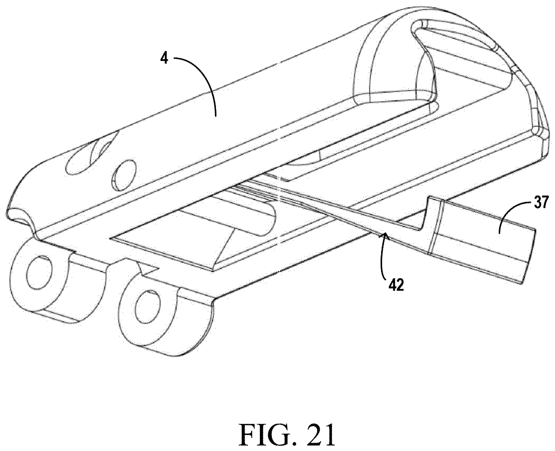

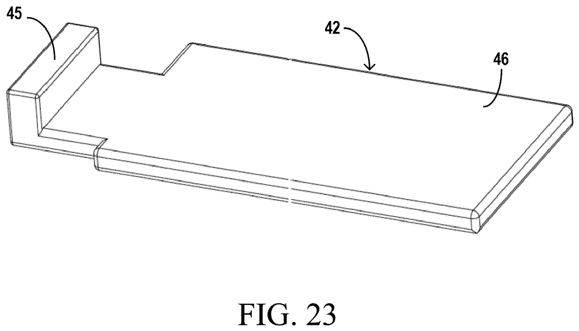

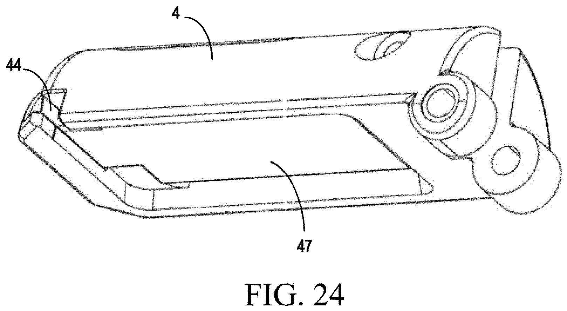

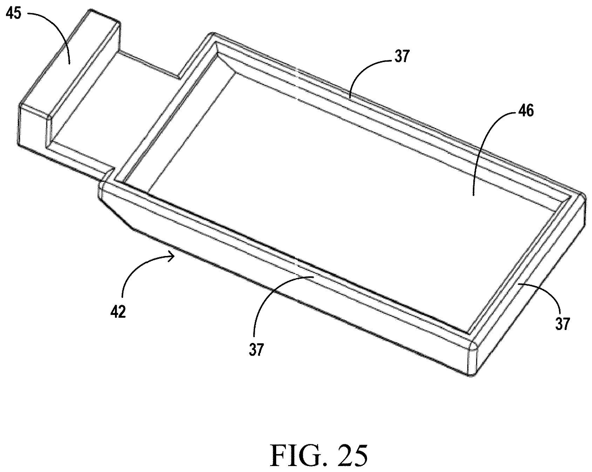

In another type of needle tip protective structure 7, with reference to FIG. 23, the needle tip protective structure 7 includes a protective surface 46 against which the needle tip of a suture needle 3 may abut during movement and when in the puncture position. Alternatively, in other types of needle tip protective structure 7, with reference to different types of needle tip protective structures shown in FIGs. 17-29, the needle tip protective structure 7 includes a protective surface 46 for the needle tip of a suture needle 3 to abut against, a protective protrusion 37 is formed on the protective surface 46, and the needle tip of the suture needle 3 is stopped at the protective protrusion 37. In this way, during the puncturing process of the suture needle 3 and at the shielding position, the needle tip of the suture needle 3 can abut against the protective surface, and the protective protrusion 37 can prevent the detachment of the suture needle 3 from the protective surface.

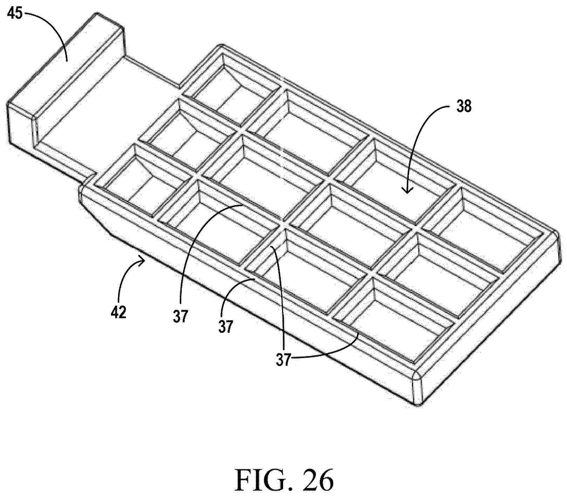

In addition, the protective protrusion 37 may have a variety of structure types. For example, in one structure type, with reference to FIGs. 17-20, a ring of protrusions is formed on the peripheral edge of the protective surface for use as the protective protrusion 37. Alternatively, in another structure type, with reference to FIG. 26, grid-like protrusions 38 are formed on the protective surface, and edges of the grid-like protrusions 38 are used as protective protrusions 37. The grid-like protrusions 38 may include transverse protrusions and vertical protrusions that are cross-arranged, or may include inclined protrusions that are obliquely cross-arranged, or may include a plurality of connected circular or oval protrusions.

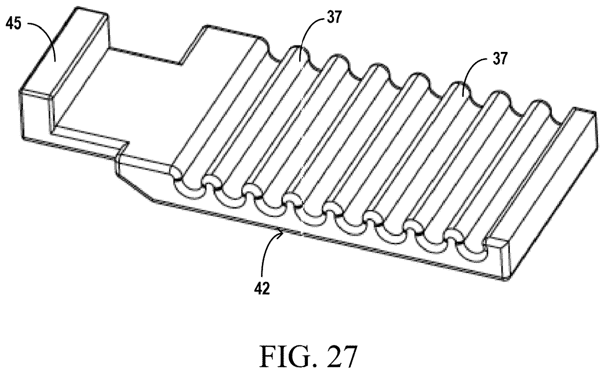

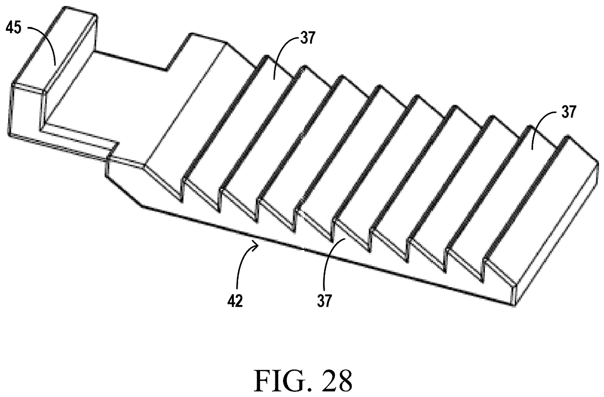

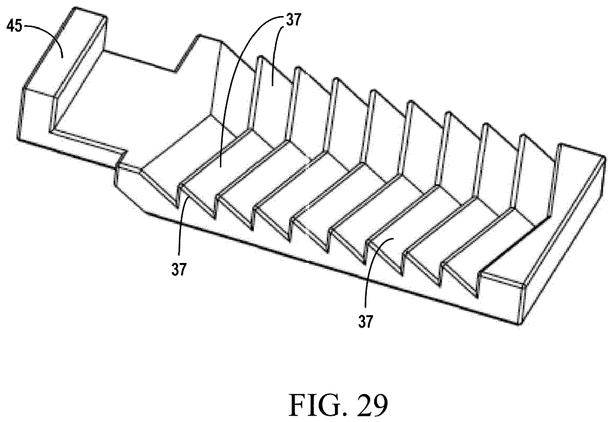

In other structural types of the protective protrusion 37, with reference to FIGs. 27-29, a plurality of protective protrusions 37 may be arranged at intervals on the protective surface in a direction away from the tube body 1. That is, the plurality of protective protrusions 37 are not arranged to cross one another, but are arranged to be spaced apart from another. For example, in some embodiments, with reference to FIGs. 27 and 28, each protective protrusion 37 extends straight, that is, each protective protrusion 37 is a straight bar shape, and the plurality of protective protrusions 37 are in a wavy arrangement or in a zigzag arrangement. In some other embodiments, with reference to FIG. 29, each protective protrusion 37 extends in a V-shaped manner, that is, each protective protrusion 37 is a V-shaped shape, and the plurality of protective protrusions 37 are in a wavy arrangement or in a zigzag arrangement.

Furthermore, in other types of needle tip protective structure 7, with reference to FIGs. 1, 17, 21, and 22, the needle tip protective structure 7 includes a plurality of stabilizers 4 and a plurality of protective plates 42 each having a protective surface (e.g., two stabilizers 4 and two protective plates 42 as shown in the figures), and the protective plates 42 are provided on the stabilizers 4 on a one-to-one basis, wherein in the puncture position, each suture needle 3 passes through a suture thread fixing portion 39 of a respective corresponding stabilizer 4 so as to flip the protective plate 42 from the retracted position to the shielding position, and wherein in the retracted position, the plurality of protective plates 42 are located inside of the respective corresponding stabilizers 4, which can ensure that there is no protrusion on the outer surfaces of the stabilizers 4, facilitating the entrance and exit of the tissue; and in the shielding position, the needle tips of the suture needles 3 are stopped at the protective protrusion 37. In this way, each suture needle 3 can drive a corresponding protective plate 42 to flip over, and during flipping, each protective plate 42 can provide good protection to the corresponding suture needle 3, avoiding exposure of the needle tip.

In addition, in some embodiments, the protective plate 42 may be an elastic body and can return from the shielding position to the retracted position under the action of its own elastic force. For example, the protective plate 42 may be a rubber plate or a silicone plate. As such, when the plurality of suture needles 3 are detached from the protective plate 42, the protective plate 42 can return to the retracted position under the action of its own elastic force.

In addition, in some embodiments, the plurality of stabilizers 4 can, when being folded, press the plurality of protective plates 42, so that the plurality of protective plates 42 return from the shielding position to the retracted position. For example, one end of each protective plate 42 may be connected on a stabilizer 4 by means of a connecting pin. In this way, when being folded, the plurality of stabilizers 4 drive the plurality of protective plates 42 to return to the retracted position.

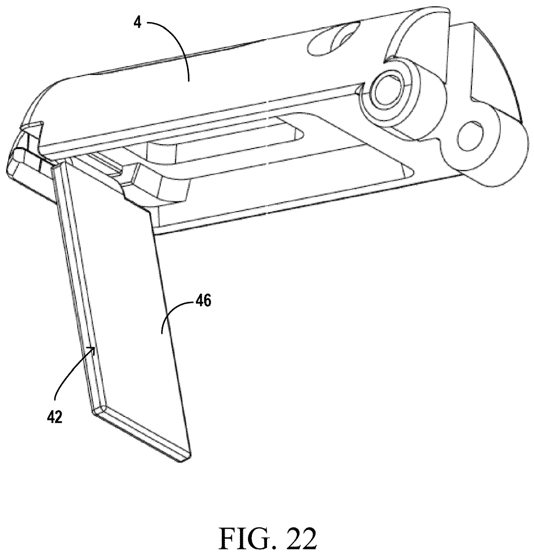

In addition, the protective plates 42 may be connected at any position of the stabilizers 4 as long as the same can provide protection against needle tips. For example, in some embodiments, with reference to FIGs. 1 and 22, the plurality of protective plates 42 are separately connected to ends of the plurality of stabilizers 4 away from the tube body 1, so that under the drive of suture needles, the plurality of protective plates 42 are unfolded outwardly away from one another to the shielding position. Alternatively, in other embodiments, with reference to FIGs. 17-18 and 21, the plurality of protective plates 42 are separately connected to the other ends of the plurality of stabilizers 4 close to the tube body 1, so that under the drive of the suture needles, the plurality of protective plates 42 are unfolded inwardly close to one another to the shielding position.

In addition, an accommodating cavity 47 may be formed on each stabilizer 4, and in the retracted position, the protective plates 42 may be located in the accommodating cavities 47. Furthermore, the protective plates 42 may be connected on the stabilizers 4 by means of a variety of connection means. For example, in one connection means, a clamping recess 44 is formed on each stabilizer 4, and a clamping edge 45 at one end of a protective plate 42 may be clamped in 44. Alternatively, in another connection means, the protective plates 42 may be connected on the stabilizers 4 by means of a support shaft or a rotary shaft.

In addition, in some embodiments, in order to further improve the protective effect of the needle tips, in the puncture position, a thread-hanging port 40 on a side surface of the plurality of suture needles 3 faces toward a protective surface. As such, the protective surface can isolate opening edges of the thread-hanging ports 40 from possible surrounding organs, which can completely prevent the opening edges of the thread-hanging ports 40 from making contact with surrounding organs that may be present, thereby improving the safety of suture needle delivery.

Furthermore, in the tissue closure device, in some embodiments, an opening, for example, an axially extending strip-shaped opening or a spiral opening, may be formed on the side wall of the tube body 1, and a manual operation portion 8 of the needle manipulator 5 may extend radially from the opening. In this way, the manual operation portion 8 may move axially and linearly along the strip-shaped opening, or the manual operation portion 8 may move along the spiral opening. Alternatively, in other embodiments, with reference to FIGs. 1 and 2, the needle manipulator 5 is disposed at a proximal end position on the tube body 1, and the manual operation portion 8 of the needle manipulator 5 extends from the proximal end port of the tube body 1. In this way, an opening may be prevented from being formed on the sidewall of the tube body 1, so that the self-strength of the tube body 1 may be improved while reducing the tube wall thickness of the tube body 1. At the time, an operator may, at the proximal end position on the tube body 1, conveniently operate the manual operation portion 8, such as pressing the manual operation portion 8 or rotating the manual operation portion 8, thereby driving a plurality of suture needles 3, for example two, to move simultaneously.

In addition, in the tissue closure device, in some embodiments, the needle manipulator 5 may be rotated. For example, the needle manipulator 5 may be rotated at the proximal end port of the tube body 1, or the needle manipulator 5 may be rotated along the spiral opening on the sidewall of the tube body 1. The needle manipulator 5 and a suture needle mounting seat may be connected by means of the cooperation of screw threads, and the suture needle mounting seat is disposed in the tube body 1 in an axially movable manner, and two suture needles 3 are connected on the suture needle mounting seat. As such, when the needle manipulator 5 is rotated forward and reversely, the manipulator can drive the suture needle mounting seat to reciprocatively and axially move, thereby driving the two suture needles 3 to move simultaneously. Alternatively, in other embodiments, with reference to FIGs. 1 and 2, the needle manipulator 5 is movable between a needle triggering position and a needle initial position in an axial direction of the tube body 1. In this way, the axial movement of the needle manipulator 5 may more quickly drive the two suturing needles 3 to move; in addition, the structure of the tissue closure device may be more simplified and compact.

In addition, in some embodiments, the outer diameter of the needle manipulator 5 may be the same as the inner diameter of the tube body 1, so that the inner surface of the tube body 1 may be in contact with the outer surface of the needle manipulator 5 in its entire circle for movement guidance. Alternatively, in some embodiments, to reduce the contact area between the tube body 1 and the needle manipulator 5, and to facilitate the needle manipulator 5 moving axially, a movement guidance structure is provided between the needle manipulator 5 and the tube body 1, and the movement guidance structure defines the needle triggering position and the needle initial position of the needle manipulator 5. In this way, the movement guidance structure may ensure that the needle manipulator 5 moves axially in a stable and reliable manner between the needle triggering position and the needle initial position. In addition, the contact area between the outer surface of the needle manipulator 5 and the inner surface of the tube body 1 is also allowed to be reduced, that is, the needle manipulator 5 and the tube body 1 make contact only by means of the movement guidance structure, and a gap remains between the needle manipulator 5 and the other portions of the tube body 1. Therefore, the needle manipulator 5 can be axially moved more easily, so as to more easily drive two suture needles 3 to extend and retract simultaneously.

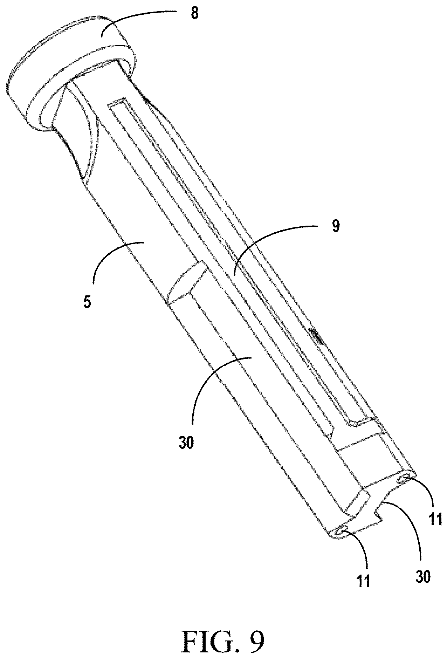

Of course, in the tissue closure device, the movement guidance structure may be of a variety of types. For example, in some types, the movement guidance structure includes two axially spaced flanges formed on the needle manipulator 5, and the two flanges make contact with the inner surface of the tube body 1. Alternatively, in other types, with reference to FIGs. 9-10, and FIGs. 13-14, the movement guidance structure comprises an axially extending guide slot 9 on the outer surface of the needle manipulator 5 and a guide bump 10 on the inner surface of the tube body 1, and the guide bump 10 is cooperates in the guide slot 9. For example, in some embodiments, one half of the guide bump 10 is formed on each of the two half tube portions 17, and the two halves of the guide bump 10 may be fitted together and cooperate in the guide slot 9. In this way, the cooperating guide bump 10 and guide slot 9 may not only ensure that the needle manipulator 5 moves axially in a stable and reliable manner, but also avoid unnecessary rotation of the needle manipulator 5. In addition, the axial length of the guide slot 9 corresponds to the needle triggering position and the needle initial position, that is, the two ends of the guide slot 9 in the axial direction define the needle triggering position and the needle initial position of the needle manipulator 5. When the guide bump 10 abuts against one end portion of the guide slot 9, the needle manipulator 5 is in the needle-triggered position. At the time, the two suture needles 3 are in the puncture position. When the guide bump 10 abuts against the other end portion of the guide slot 9, the needle manipulator 5 is in the needle initial position. At the time, the two suture needles 3 are retracted into the tube body 1. For example, the needles are exactly located in the respective needle outlet channels 2.

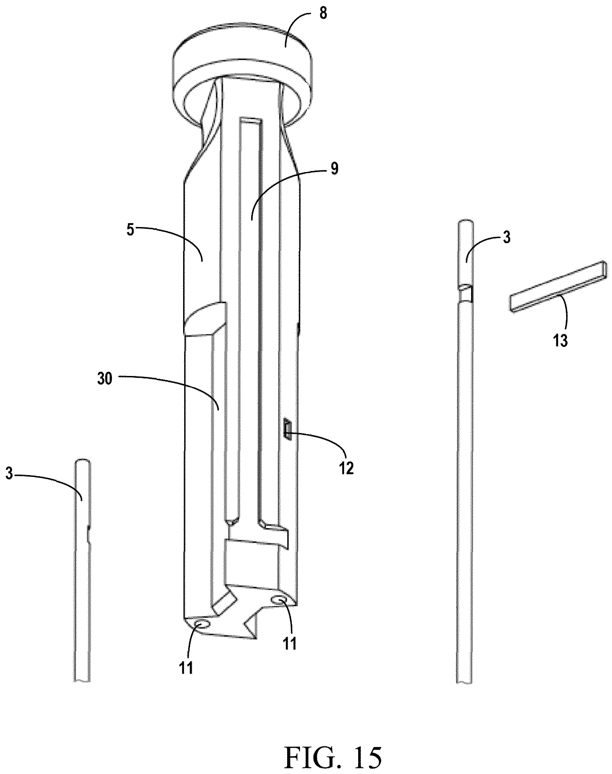

In addition, in the tissue closure device, in some embodiments, two suture needles 3 may be connected to the needle manipulator 5 by means of an intermediate transition piece, e.g., the suture needle mounting seat as previously mentioned. Alternatively, in other embodiments, with reference to FIG. 15, a plurality of suture needles 3, e.g., two suture needles 3, are directly connected to the needle manipulator 5. For example, a mounting section of one end of a suture needle 3 has a position-limiting surface, and the mounting section may be directly inserted in a position-limiting hole of the needle manipulator 5. In this way, the assembly of the two suture needles 3 and the needle manipulator 5 may be facilitated, thereby improving the convenience of assembly, and the needle manipulator 5 more accurately driving the two suture needles 3 to simultaneously move is also facilitated.

In addition, in the tissue closure device, a plurality of suture needles (e.g., two suture needles 3) may be connected to the needle manipulator 5 by using a variety of means. For example, in some means, a threaded section is formed at one end of a suture needle 3, and the threaded section can be threadedly connected to a threaded hole on the needle manipulator 5. Alternatively, in some means, with reference to FIG. 15, a plurality of radially spaced needle insert holes 11, e.g., two radially spaced needle insert holes 11, are formed on a distal end surface of the needle manipulator 5; the plurality of needle insert holes 11, e.g., two needle insert holes 11, extend toward a proximal end of the needle manipulator 5; a radial hole 12 in communication with the plurality of needle insert holes 11 are formed on the needle manipulator 5; and a locking pin 13 is inserted into the radial hole 12, wherein ends of the plurality of suture needles 3 are inserted in the respective needle insert holes 11, and are lock-fitted with the locking pin 13 at the same time. For example, in some embodiments, one locking pin 13 is simultaneously lock-fitted with ends of two suture needles 3.

In addition, in some embodiments, with reference to FIGs. 3 and 4, the needle manipulator 5 allows the driver 6 to move between the stabilizer triggering position and the stabilizer initial position when in the needle initial position, and the needle manipulator 5 blocks the driver 6 from returning from the stabilizer triggering position to the stabilizing initial position when in the needle triggering position. As such, when the suture needles are not retracted into the tube body 1, it is possible to prevent the unexpected retraction of the stabilizers 4 from causing the suture needles to deform and damage other organs that may be present in the periphery, thereby improving safety. Therefore, only when the needle manipulator 5 is moved from the needle triggering position to the needle initial position so that the suture needles are fully retracted into the tube body 1, can the driver 6 move from the stabilizer triggering position to the stabilizer initial position so that the stabilizers 4 move from the unfolded position to the folded position.

In addition, in the tissue closure device, similar to the needle manipulator 5, the driver 6 may be configured to rotate. For example, the driver 6 can be rotated at the proximal end port of the tube body 1, or the driver 6 can be rotated along the spiral opening on the sidewall of the tube body 1. The driver 6 is connected to a connecting rod or a driving rod by means of the cooperation of screw threads, and the connecting rod or the driving rod is disposed in the tube body 1 in an axially movable manner and is connected to the stabilizers 4. As such, when the driver 6 is rotated forward and reversely, the driver may drive the connecting rod or the driving rod to reciprocatively and axially move, thereby driving the stabilizers 4 to unfold and fold.

Alternatively, in some embodiments, with reference to FIGs. 1 and 2, the driver 6 is movable between the stabilizer initial position and the stabilizer triggering position in an axial direction of the tube body 1, so that the axial movement of the driver 6 may more quickly drive the stabilizers to unfold and fold; in addition, the structure of the tissue closure device may be more simplified and compact.

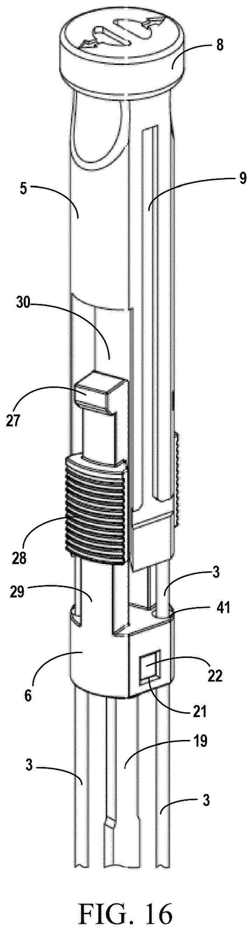

In addition, in some embodiments, with reference to FIG. 16, a plurality of radially spaced axial channels 41 are formed on the driver 6, and the plurality of suture needles 3 pass through respective corresponding axial channels 41. In this way, the internal space of the tube body 1 may be fully utilized to arrange the driver 6, the needle manipulator 5 and the plurality of suture needles 3, so that the volume of the tube body 1 is smaller, and the internal structure of the tissue closure device is more compact.

In addition, the driver 6 may be directly connected to one or more stabilizer 4. Alternatively, with reference to FIG. 16, the tissue closure device further includes a driving rod 19 located in the tube body 1, a proximal end of the driving rod 19 is directly connected to the driver 6, and a distal end of the driving rod 19 extends from a distal end of the tube body 1 so as to connect a plurality of stabilizers 4. In this way, because the driving rod 19 is utilized, the volume of the driver 6 may be effectively reduced, and assembly of the driving rod 19 may be easier.

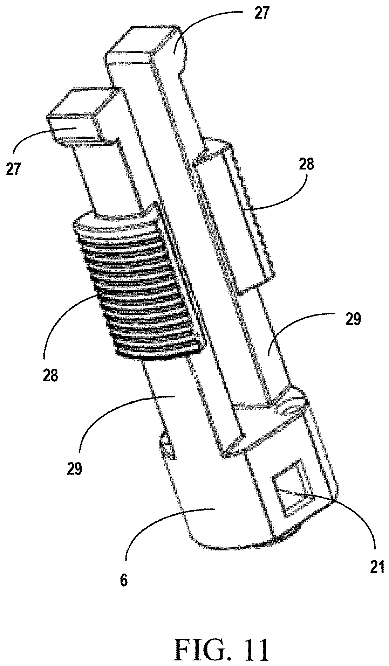

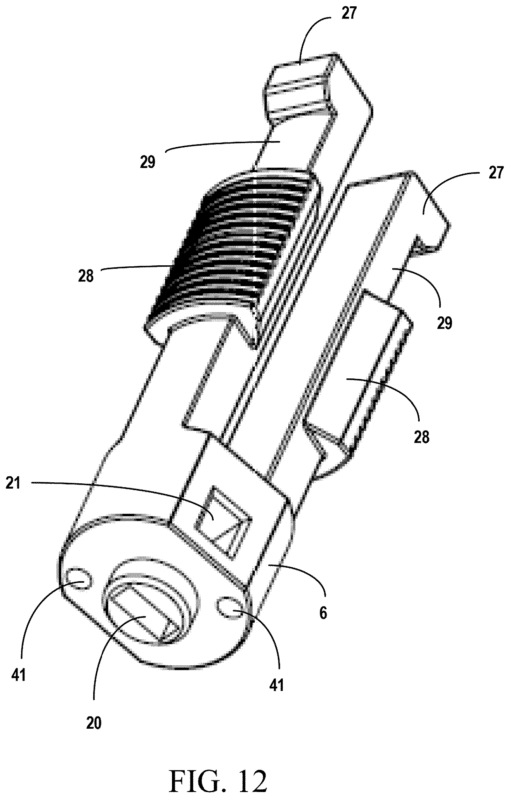

In addition, the driver 6 and the driving rod 19 may be welded. Alternatively, they may be thermally fused. Alternatively, in some embodiments, with reference to FIGs. 11 and 12, an axially extending central hole 20 is formed on a distal end surface of the driver 6, a radial channel 21 in communication with the central hole 20 is formed on the driver 6, and a locking shaft 22 is provided in the radial channel 21, wherein the proximal end of the driving rod 19 is inserted in the central hole 20 and lock-fitted with the locking shaft 22.

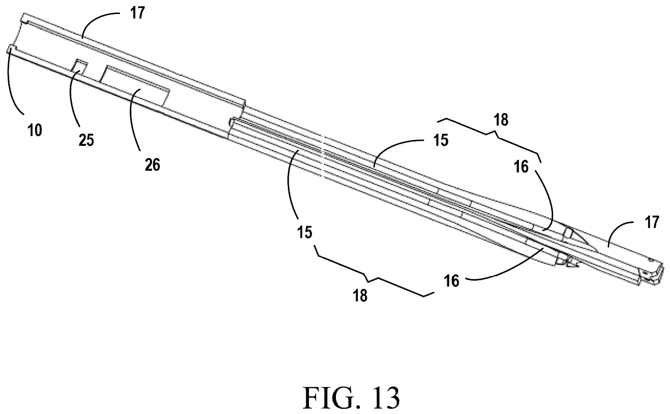

In addition, in some embodiments, for ease of assembly of the tissue closure device, with reference to FIGs. 13 and 14, the tube body 1 includes two half tube portions 17 that can be fitted together. A half-driving-rod channel recess 23 is formed on each of the half tube portions 17. When the two half tube portions 17 are combined to form the tube body 1, the half-driving-rod channel recess 23 on one of the half tube portions 17 and the half-driving-rod channel recess 23 on the other of the half tube portions 17 are fitted so as to form a driving rod accommodating channel 24, wherein the driving rod 19 is axially and movably disposed in the driving rod accommodating channel 24. For example, in some embodiments, in each half tube portion 17, the half-driving-rod channel recess 23 is located between two fitting surfaces.

In addition, in some embodiments, with reference to FIG. 8, the two half tube portions 17 may be completely identical. For example, each half tube portion 17 may include two fitting surfaces that are arranged at an interval and has a height difference, a suture needle channel recess 18 is formed on each fitting surface, and a part between the two fitting surfaces may be used as one half-driving-rod channel recess 23. In this way, when two identical half tube portions 17 are fitted, the fitting surfaces are connected in a form-fitting manner, so that the suture needle channel recesses 18 form suture needle channels 14. The two suture needle channel recesses 18 are mutually fitted to form a driving rod accommodating channel 24, as shown in FIG. 8.

In addition, in some embodiments, with reference to FIGs. 1 and 13, an engagement opening 25 and a driving opening 26 which are axially spaced are formed on the sidewall of the tube body 1, wherein the driver 6 includes a fastener 27 and a driving portion 28 which are axially spaced, wherein during the axial movement of the driver 6, the driving portion 28 is always located in the driving opening 26; wherein when the fastener 27 is engaged in the engagement opening 25, the driver 6 is in the stabilizer initial position; and wherein when the fastener 27 is engaged in the driving opening 26, the driver 6 is in the stabilizer triggering position. As such, an operator presses the driving portion 28 so that after the fastener 27 is moved out from the engagement opening 25, the driver 6 may then move from the stabilizer initial position to the stabilizer triggering position. Likewise, the operator presses the driving portion 28 so that after the fastener 27 is moved out from the driving opening 26, the driver 6 may then move from the stabilizer triggering position to the stabilizer initial position.

In addition, in some embodiments, the operator may operate the driving portion 28 so that the driver 6 moves between the stabilizer initial position and the stabilizer triggering position. Alternatively, in other embodiments, the driver 6 is connected to an elastic member, such as a spring 43. When the driver 6 moves from one among the stabilizer initial position and the stabilizer triggering position to the other, the elastic member may be in an energy storage state. When the elastic member releases energy, the same may drive the driver 6 to return easily.

In addition, as described above, when in the needle triggering position, the needle manipulator 5 blocks the driver 6 from returning from the stabilizer triggering position to the stabilizer initial position. At the time, when in the needle triggering position, the needle manipulator 5 blocks the fastener 27 from being detached from the driving opening 26, so as to block the driver 6 from returning from the stabilizer triggering position to the stabilizer initial position.

In addition, in some embodiments, in order to improve the convenience of the pressing operation of the driving portion 28, with reference to FIGs. 11 and 12, the driver 6 includes a suspension arm 29, and the fastener 27 and the driving portion 28 are disposed on the suspension arm 29, and the fastener 27 is located at a proximal end of the suspension arm 29. As such, by means of the suspension arrangement of the suspension arm 29, the operator applies a reduced force to the driving portion 28, so that the fastener 27 may be moved out from the engagement opening 25 and the driving opening 26.

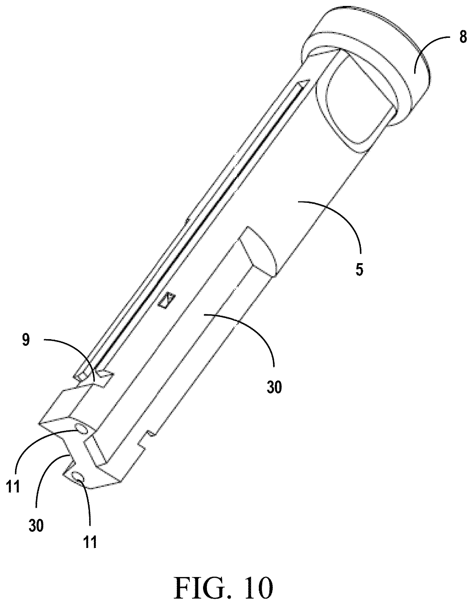

In addition, in some embodiments, with reference to FIGs. 9 and 10, an axially extending guide recess 30 is formed on the needle manipulator 5, wherein when the needle manipulator 5 is axially moved to the needle initial position, the suspension arm 29 is located on the exterior of the guide recess 30; and wherein when the needle manipulator 5 is axially moved to the needle triggering position, the suspension arm 29 is slid into the guide recess 30. As such, by means of the guide fit between the guide recess 30 and the suspension arm 29, the needle manipulator 5 may move axially in a more stable and reliable manner, so as to drive the plurality of suture needles to move more smoothly. At the same time, when the suspension arm 29 is slid into the guide recess 30, the guide recess 30 may block the suspension arm 29 from moving radially inwardly. In this way, the fastener 27 may be blocked from moving out from the driving opening 26, thereby blocking the driver 6 from returning from the stabilizer triggering position to the stabilizer initial position.

In addition, the number of suspension arms 29 and guide recesses 30 may be selected according to actual requirements. For example, the number of suspension arms 29 and guide recesses 30 is one respectively. Alternatively, the number of suspension arms 29 and guide recesses 30 may be multiple, such as two, three, or four. For example, in some embodiments, with reference to FIG. 16, two suspension arms 29 and two guide recesses 30 are provided and are respectively arranged at intervals in a radial direction, and the suspension arms 29 correspond one-to-one to the guide recesses 30, wherein when the needle manipulator 5 is in the needle triggering position, a distal end of the needle manipulator 5 is between the two suspension arms 29.

In addition, in some embodiments, with reference to FIG. 14, the tube body 1 includes two half tube portions 17 that can be fitted together, and the engagement opening 25 and the driving opening 26 are formed on each of the half tube portions 17. In this way, the assembly of the needle manipulator 5 and the driver 6 may be facilitated by means of fitting the two half tube portions 17.

In addition, in the tissue closure device, suture threads may be located on the exterior of the tube body 1. Two ends of each suture thread may be arranged in thread-hanging ports 40 of a suture needle by using a variety of means. For example, in some embodiments, when wiring, the suture needle may be pushed out slightly from the needle outlet channel 2, the two ends of each suture thread are then hooked and positioned in the thread-hanging ports 40, and then the suture needle is retracted into the needle outlet channel 2. When puncturing and wiring, the suture needle carries the suture thread to pass through tissue and the suture thread fixing portion 39 on the stabilizer and then moves to the puncture position. At the time, the suture thread fixing portion 39 holds and positions the suture thread. As such, when the suture thread is withdrawn, the suture thread will remain on the suture thread fixing portion 39. Then, the stabilizer is moved to the folded position and the two ends of the suture thread are drawn out from the tissue for suturing.

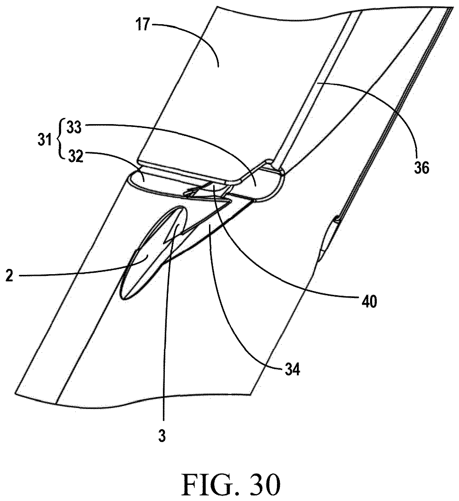

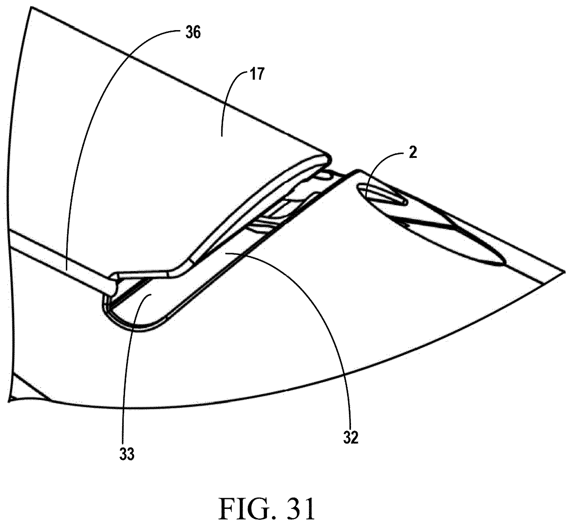

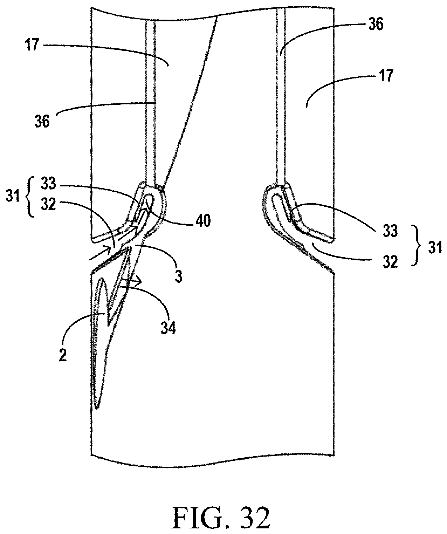

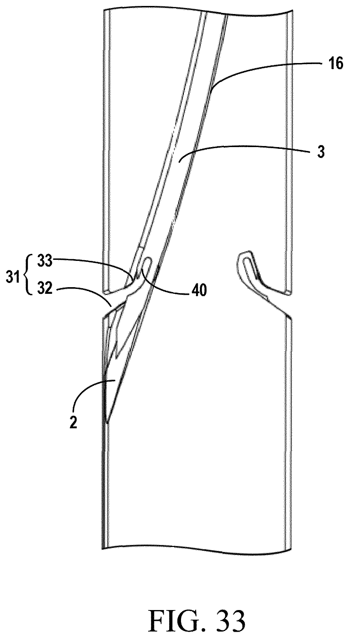

Alternatively, in some other embodiments, in order to improve the convenience of wireloading, with reference to FIGs. 30-35, at each needle outlet channel 2, a wiring notch 31 in communication with the needle outlet channel 2 is formed on the tube body 1, wherein two of the wiring notches 31 are used to accommodate at least two ends of a suture thread located on the exterior of the tube body 1, respectively; and wherein, when the needle manipulator 5 is in the needle initial position, a thread-hanging port 40 of each suture needle is at least partially in communication with the wiring notch 31 so that the suture thread can enter the thread-hanging port 40 via the wiring notch 31, that is, the wiring notches 31 are configured such that at least two ends of the suture thread entering the wiring notches 31 are located in two of the thread-hanging ports 40, respectively. The process of the suture thread entering the wiring notch 31 and the thread-hanging port 40 is shown by three arrows in FIG. 32. In this way, in the process of arranging the suture threads in the thread-hanging ports, the suture needles are pre-arranged in the inside of the tube body 1, so the suture needles are not exposed, which is safer in terms of the operation of the operator; in addition, the suture needles can also be prevented from being polluted by the outside. In this way, the operator may directly arrange two ends of the suture thread, e.g., two ends of approximately 3-5 cm of the suture thread, in the wiring notches 31. At the time, the two ends of the suture thread will be located in the thread-hanging ports 40 of a suture needle. At the time, as described above, when puncturing and wiring, the suture needle carries the suture thread to pass through tissue and the suture thread fixing portion 39 on the stabilizer and then moves to the puncture position. At the time, the suture thread fixing portion 39 holds and positions the suture thread. In this way, when the suture thread is withdrawn, the suture thread will remain on the suture thread fixing portion 39. Then, the stabilizer is moved to the folded position and the two ends of the suture thread are drawn out from the tissue for suturing.

**In** some embodiments, in order to improve the reliability and smoothness of wiring, the inner walls of the wiring notches 31 may have a plane, and the suture needles 3 may be placed against the plane so that the thread-hanging ports 40 and the wiring notches 31 are at least partially in communication. **In** this way, the twisting of the suture needles 3 may be avoided so that the thread-hanging ports 40 and the wiring notches 31 are not misaligned and the suture threads cannot enter.

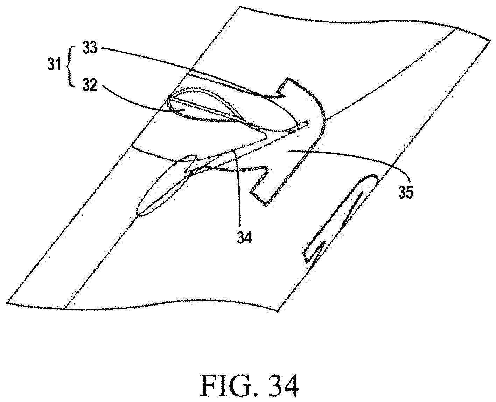

**In** addition, in order to improve the stability and reliability of wiring, with reference to FIGs. 32 and 34, each wiring notch 31 includes an inlet section 32 and a clamping slit 33, wherein the size of the clamping slit 33 is less than that of the inlet section 32, so that a suture thread entering the clamping slit 33 may be clamped in the clamping slit 33. When the needle manipulator 5 is in the needle initial position, the clamping slit 33 is configured such that at least two ends of the suture thread clamped in the clamping slits 33 are located in two of the thread-hanging ports 40, respectively. **In** this way, the operator may directly lead the two ends of the suture thread, for example, the two ends of about 3-5 cm of the suture thread, through the inlet sections 32 into the clamping slits 33 separately so that the two ends are clamped by the clamping slits 33. At the time, the two ends of the suture thread will be located in the thread-hanging ports 40 of the suture needle. At the time, as described above, when puncturing and wiring, the suture needle carries the suture thread to pass through tissue and the suture thread fixing portion 39 on the stabilizer and then moves to the puncture position. At the time, the suture thread fixing portion 39 holds and positions the suture thread. **In** this way, when the suture thread is withdrawn, the suture thread will remain on the suture thread fixing portion 39. Then, the stabilizer is moved to the folded position and the two ends of the suture thread are drawn out from the tissue for suturing.

In addition, in order to further improve the convenience of wiring, with reference to FIG. 34, the size of the inlet section 32 gradually decreases from the exterior to the inside. In this way, the operator may more conveniently lead the two ends of the suture thread into the clamping slits 33 along the inlet ends 32.

In addition, in some embodiments, with reference to FIGs. 32 and 34, each wiring notch 31 and the needle outlet channel 2 corresponding thereto are arranged in an intersecting manner. For example, the wiring notches 31 are arranged approximately at the middle position of the needle outlet channels 2. At the time, at one end of each wiring notch 31, a thread-withdrawing notch 34, which communicates an outlet end of a needle outlet channel 2 with the one end of the wiring notch 31, is formed on the tube body 1. The thread-withdrawing notch 34 is used to allow the suture thread, which is drawn out when the suture needle 3 is extending obliquely from the needle outlet channel 2, to withdraw from the needle outlet channel 2. As shown by the single arrow in FIG. 32, the suture thread can be withdrawn from the needle outlet channels 2 through the thread-withdrawing notch 34. As such, when puncturing and wiring, the suture needle carries the suture thread to extend obliquely from the needle outlet channel 2 and pass through tissue and the suture thread fixing portion 39 on the stabilizer and then moves to the puncture position. At the time, the suture thread may be withdrawn from the thread-withdrawing notch 34.

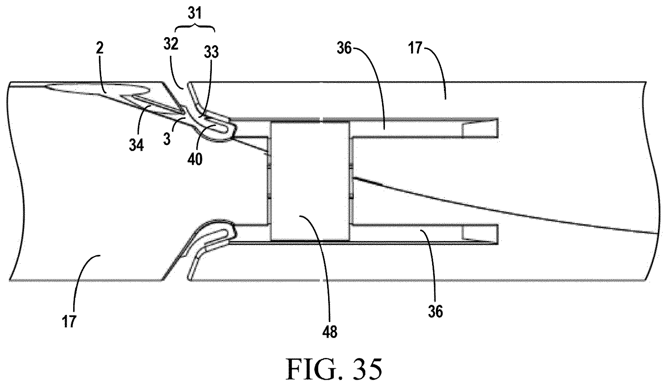

In addition, in some embodiments, the wiring notches 31 and the thread-withdrawing notches 34 may be formed directly on the sidewall of the tube body 1. Alternatively, in other embodiments, with reference to FIG. 35, the needle outlet channels 2, the wiring notches 31, and the thread-withdrawing notches 34 may be formed on a single component, and the single component may be mounted on the tube body 1. For example, hollow notches are formed on the tube body 1, and an elastic block 35 is provided in each hollow notch, wherein the needle outlet channels 2, the wiring notches 31, and the thread-withdrawing notches 34 are formed on the elastic blocks 35. The elastic blocks 35 may be rubber blocks or silicone blocks. As such, the self-elasticity of the elastic blocks 35 may be utilized so that it is easier for the wiring notches 31 (e.g., the clamping slits 33) to position the two ends of a suture thread.

In addition, in some embodiments, in order to improve the orderliness of the suture thread on the exterior of the tube body 1, with reference to FIGs. 30-35, a wiring structure is formed on the outer surface of the tube body 1, and the wiring structure is used to position the suture thread located on the exterior of the tube body 1. As such, by means of the wiring structure, the suture thread may be orderly arranged on the outer surface of the tube body 1 to avoid knotting of the suture thread. Of course, during the puncture and thread-introducing process, the suture thread may be detached from the wiring structure due to the pulling of suture needles against the suture thread.

Of course, the wiring structure may have a variety of forms. For example, in one form, the wiring structure includes one or more detachable thread-pressing blocks 48 disposed on the outer surface of the tube body 1. At the time, the thread-pressing block(s) 48 may press the suture thread on the outer surface of the tube body 1. Alternatively, in another form, the wiring structure includes wiring slots 36 that extend toward the proximal end of the tube body 1 and that are formed on the outer surface of the tube body 1, and the wiring slots 36 are used to accommodate the suture thread located on the exterior of the tube body 1. In this way, the suture thread on the exterior of the tube body 1 may be located in the wiring slots 36 along the extending trajectory of the wiring slots 36.

The wiring slots 36 may be U-shaped, V-shaped or have other possible shapes.

In addition, in some embodiments, the wiring slots 36 may not communicate with two wiring notches 31 of the tube body 1 that are in communication with two of the needle outlet channels 2, so that a portion of the suture thread will extend out of the outer surface of the tube body 1. Alternatively, in other embodiments, with reference to FIG. 32, the wiring slots 36 communicate with two wiring notches 31 of the tube body 1 that are in communication with two of the needle outlet channels 2, so that a portion of the suture thread located between the two wiring notches 31 may be completely located in the wiring slots 36.

In addition, in some embodiments, when the tube body 1 is divided into two half tube portions 17 (e.g., a first half tube portion and a second half tube portion), the suture needles 3, the needle manipulator 5, the driver 6, and the driving rod 19 of the tissue closure device may be disposed on one half tube portion 17 (the first half tube portion), and the other half tube portion 17 (the second half tube portion) is fitted on the one half tube portion 17 so that the suture needle 3, the needle manipulator 5, the driver 6, and the driving rod 19 are disposed in one step in the tube body 1, wherein the driver 6 drives the stabilizers 4 at the distal end of the tissue closure device to fold and unfold by means of the driving rod 19, and thus the convenience of assembling the tissue closure device can be significantly improved.

Also disclosed is a tissue closing method which is not explicitly claimed but which may be performed using tissue closure devices according to the invention. The tissue closing method includes: arranging a suture thread on the exterior of a tube body of a tissue closure device, and positioning at least two ends of the suture thread at two needle outlet channels of the tube body, respectively; unfolding, by means of a driver on the tube body, stabilizers in an inner cavity of tissue to be sutured, and abutting the stabilizers against an inner cavity wall of the tissue; driving, by means of a needle manipulator, a plurality of suture needles in the tube body to simultaneously extend obliquely from corresponding needle outlet channels such that two ends of the suture thread are drawn to pass through the tissue to be sutured and suture thread fixing portions of the stabilizers and then move to a puncture position; driving, by means of the needle manipulator, the plurality of suture needles back into the tube body, and retaining the two ends of the suture thread on the corresponding suture thread fixing portions, respectively; and folding the stabilizers by means of the driver on the tube body to draw out the two ends of the suture thread from a wound to be sutured.

In said tissue closing method, since the needle manipulator can drive the plurality of suture needles to simultaneously move, so as to simultaneously extend obliquely from the needle outlet channels and pass through tissue to move to the puncture position, as such, the needle manipulator can drive in one step the plurality of suture needles to the puncture position, so that the consistency and symmetry of the puncture position can be improved, and deviation of the puncture position can be avoided, thereby improving the safety of surgical suturing.

In addition, in some embodiments, at least two ends of the suture thread are arranged in wiring notches on the tube body that are in communication with needle outlet channels, so that the at least two ends of the suture thread entering the wiring notches are located in thread-hanging ports of two suture needles, respectively. In this way, the operator may directly arrange two ends of the suture thread, e.g., two ends of approximately 3-5 cm of the suture thread, in the wiring notch. At the time, the two ends of the suture thread will be located in the thread-hanging ports of the suture needle. At the time, when puncturing and wiring, the suture needles carry the suture thread to pass through the tissue and the suture thread fixing portions on stabilizers and then move to the puncture position. At the time, the suture thread fixing portions clamp and position the suture thread. In this way, when the suture thread is withdrawn, the suture thread will remain on the suture thread fixing portions. Then, the stabilizers are moved to the folded position and the two ends of the suture thread are drawn out from the tissue for suturing.

Furthermore, in some embodiments, at least two ends of the suture thread are arranged in clamping slits of the wiring notches, so that the at least two ends of the suture thread clamped in the clamping slits are located in two of the thread-hanging ports, respectively. In this way, the operator may directly lead the two ends of the suture thread, for example, the two ends of about 3-5 cm of the suture thread, into the clamping slits separately so that the two ends are clamped by the clamping slits. At the time, the two ends of the suture thread will be located in the thread-hanging ports of the suture needle. When puncturing and wiring, the suture needle carries the suture thread to pass through tissue and the suture thread fixing portion on a stabilizer and to then move to the puncture position. At the time, the suture thread fixing portion clamps and positions the suture thread. In this way, when the suture thread is withdrawn, the suture thread will remain on the suture thread fixing portion. Then, the stabilizer is moved to the folded position and the two ends of the suture thread are drawn out from the tissue for suturing.

In addition, in some embodiments, after the plurality of suture needles respectively pass through the suture thread fixing portions of the stabilizer, the plurality of suture needles drives a needle tip protective structure of the tissue closure device from a retracted position to a shielding position, and during the process of driving from the retracted position to the shielding position, and in the shielding position, the needle tip protective structure shields needle tips of the plurality of suture needles. In this way, the needle tip protective structure is driven to the shielding position by means of the plurality of suture needles, so that the needle tips of the suture needles may always remain in contact with the needle tip protective structure in the process of moving the suture needles through tissue toward the puncture position. Thus, it may effectively prevent the needle tips of the suture needles from slipping or detaching from the needle tip protective structure and causing damage to surrounding organs, thereby improving the safety of surgical suturing.

In addition, in some embodiments, the needle manipulator drives the plurality of suture needles to be in the puncture position, and blocks the driver from folding the stabilizers. In this way, when the suture needles are not retracted into the tube body, it is possible to prevent the unexpected folding of the stabilizers from causing the suture needles to deform and thus damage other organs that may be present in the periphery, thereby improving safety. Therefore, only when the needle manipulator is moved from the needle triggering position to the needle initial position so that the plurality of suture needles are fully retracted into the tube body, can the driver move from the stabilizer triggering position to the stabilizer initial position so that the stabilizers move from the unfolded position to the folded position.

It should be understood by those skilled in the art that the above embodiments are all exemplary and not restrictive. Different technical features in different embodiments can be combined to achieve beneficial effects. On the basis of studies on the attached drawings, the specification and the claims, those skilled in the art should be able to understand and implement other variations of the disclosed embodiments. Any reference signs in the claims should not be construed as limiting the scope of protection. The appearance of certain technical features in different dependent claims does not mean that these technical features cannot be combined to obtain beneficial effects.

## Claims

1. A tissue closure device, wherein the device comprises:
a tube body (1) in which a plurality of needle outlet channels (2) are formed;
a plurality of suture needles (3) located in the tube body (1);
a needle manipulator (5) disposed on the tube body (1) and movable between a needle initial position and a needle triggering position; and
stabilizers (4) and a driver (6);
wherein the needle manipulator (5) can drive the plurality of suture needles (3) to simultaneously move so as to simultaneously extend obliquely from respective corresponding needle outlet channels (2) to a puncture position;
wherein the needle manipulator (5) allows the plurality of suture needles (3) to simultaneously move so as to simultaneously retract into the respective corresponding needle outlet channels (2);
wherein the stabilizers (4) are disposed on the tube body (1) and movable between an unfolded position and a folded position;
wherein the driver (6) is disposed in the tube body (1) and movable between a stabilizer initial position and a stabilizer triggering position;
wherein the driver (6) can drive the stabilizers (4) to move, and in the stabilizer initial position, the stabilizers (4) are in the folded position, and in the stabilizer triggering position, the stabilizers (4) are in the unfolded position;
wherein, when in the needle initial position, the needle manipulator (5) allows the driver (6) to move between the stabilizer triggering position and the stabilizer initial position; and **characterized in that**:
when in the needle triggering position, the needle manipulator (5) blocks the driver (6) from returning from the stabilizer triggering position to the stabilizer initial position.

2. The tissue closure device according to claim 1, wherein a plurality of independent suture needle channels (14) are provided in the tube body (1), each of the suture needles (3) is movably located in a respective suture needle channel (14), and each of the suture needle channels (14) comprises a curvilinear channel section to guide the plurality of suture needles (3) so that the needles can simultaneously extend obliquely from the respective corresponding needle outlet channels (2) to the puncture position.

3. The tissue closure device according to claim 2, wherein each of the suture needle channels (14) comprises a rectilinear channel section and the curvilinear channel section; and optionally wherein:
in a radial direction of the tube body (1), the rectilinear channel section is located on one side of the tube body (1), the curvilinear channel section extends from the one side of the tube body (1) to the other side, and one end of the curvilinear channel section serves as the needle outlet channel (2).

4. The tissue closure device according to claim 2, wherein the tube body (1) comprises two half tube portions (17) that can be fitted together, a plurality of mutually-independent half-suture-needle channel recesses (18) are formed on each of the half tube portions (17), and when the two half tube portions (17) are combined to form the tube body (1), the plurality of half-suture-needle channel recesses (18) on one of the half tube portions (17) and the plurality of half-suture-needle channel recesses (18) on the other of the half tube portions (17) are fitted respectively, so as to form the plurality of suture needle channels (14); and optionally wherein
each of the suture needle channel recesses (18) comprises a rectilinear recess section (15) located on one side in a radial direction of the tube body (1) and a curvilinear recess section (16) extending from the one side to the other side.

5. The tissue closure device according to claim 1, wherein the tissue closure device comprises a needle tip protective structure (7) having a retracted position and a shielding position, wherein the suture needles (3) can drive the needle tip protective structure (7) from the retracted position to the shielding position, and during the process of driving from the retracted position to the shielding position, and in the shielding position, the needle tip protective structure (7) shields needle tips of the suture needles (3).

6. The tissue closure device according to claim 5, wherein the needle tip protective structure (7) comprises a protective surface for the needle tips of the suture needles (3) to abut against, protective protrusions (37) are formed on the protective surface, and in the puncture position, the needle tips of the suture needles (3) are stopped at the protective protrusions (37).

7. The tissue closure device according to claim 6, wherein grid-like protrusions (38) are formed on the protective surface, and edges of the grid-like protrusions (38) serve as the protective protrusions (37).

8. The tissue closure device according to claim 6, wherein the plurality of protective protrusions (37) are arranged at intervals on the protective surface in a direction away from the tube body (1); and optionally wherein:
each of the protective protrusions (37) extends straight, and the plurality of protective protrusions (37) are in a wavy arrangement or in a zigzag arrangement; or
each of the protective protrusions (37) extends in a V-shaped manner, and the plurality of protective protrusions (37) are in a wavy arrangement or in a zigzag arrangement.

9. The tissue closure device according to claim 6, wherein the needle tip protective structure (7) comprises the plurality of stabilizers (4) and a plurality of protective plates (42) that each have the protective surface, the protective plates (42) are provided on the stabilizers (4) on a one-to-one basis, and in the puncture position, each of the suture needles (3) passes through a suture thread fixing portion (39) of the respective corresponding stabilizer (4) to flip the protective plate (42) from the retracted position to the shielding position, wherein in the retracted position, the plurality of protective plates (42) are located inside the respective corresponding stabilizers (4), and in the shielding position, the needle tips of the suture needles (3) are stopped at the protective protrusions (37).

10. The tissue closure device according to claim 9, wherein either:
the protective plate (42) is an elastic body and can return from the shielding position to the retracted position under its own elastic force;
the plurality of stabilizers (4) can, when being folded, press the plurality of protective plates (42), so that the plurality of protective plates (42) return from the shielding position to the retracted position; or
the plurality of protective plates (42) are connected to ends of the plurality of stabilizers (4) away from the tube body (1) respectively, or the plurality of protective plates (42) are connected to the other ends of the plurality of stabilizers (4) close to the tube body (1) respectively.

11. The tissue closure device according to claim 6, wherein thread-hanging ports (40) on side surfaces of the plurality of suture needles (3) are oriented toward the protective surface in the puncture position.

12. The tissue closure device according to claim 1, wherein the needle manipulator (5) is disposed at a proximal end position on the tube body (1), and a manual operation portion (8) of the needle manipulator (5) extends from a proximal end port of the tube body (1).

13. The tissue closure device according to claim 1, wherein the needle manipulator (5) is movable between the needle initial position and the needle triggering position in an axial direction of the tube body (1).

14. The tissue closure device according to claim 13, wherein a movement guidance structure is provided between the needle manipulator (5) and the tube body (1), the movement guidance structure defining the needle initial position and the needle triggering position of the needle manipulator (5); and optionally wherein:
the movement guidance structure comprises an axially extending guide slot (9) on an outer surface of the needle manipulator (5) and a guide bump (10) on an inner surface of the tube body (1), and the guide bump (10) is fitted in the guide slot (9).

15. The tissue closure device according to claim 13, wherein a plurality of radially spaced needle insert holes (11) are formed on a distal end surface of the needle manipulator (5), the plurality of needle insert holes (11) extend toward a proximal end of the needle manipulator (5), a radial hole (12) in communication with the plurality of needle insert holes (11) is formed on the needle manipulator (5), and a locking pin (13) is inserted into the radial hole (12), wherein ends of the plurality of suture needles (3) are inserted into the respective needle insert holes (11) and at the same time are lock-fitted with the locking pin (13).

16. The tissue closure device according to claim 1, wherein the driver (6) is movable between the stabilizer initial position and the stabilizer triggering position in an axial direction of the tube body (1).

17. The tissue closure device according to claim 16, wherein a plurality of radially spaced axial channels are formed on the driver (6), and the plurality of suture needles (3) pass through the respective corresponding axial channels, respectively.

18. The tissue closure device according to claim 16, wherein the tissue closure device further comprises a driving rod (19) located in the tube body (1), a proximal end of the driving rod (19) is directly connected to the driver (6), and a distal end of the driving rod (19) extends from a distal end of the tube body (1) to connect to the plurality of stabilizers (4).

19. The tissue closure device according to claim 18, wherein an axially extending central hole (20) is formed on a distal end surface of the driver (6), a radial channel (21) in communication with the central hole (20) is formed on the driver (6), and a locking shaft (22) is provided in the radial channel (21), wherein the proximal end of the driving rod (19) is inserted in the central hole (20) and lock-fitted with the locking shaft (22).

20. The tissue closure device according to claim 18, wherein the tube body (1) comprises two half tube portions (17) that can be fitted together, a half-driving-rod channel recess (23) is formed on each of the half tube portions (17), when the two half tube portions (17) are combined to form the tube body (1), the half-driving-rod channel recess (23) on one of the half tube portions (17) and the half-driving-rod channel recess (23) on the other of the half tube portions (17) are fitted so as to form a driving rod accommodating channel (24), wherein the driving rod (19) is axially movably disposed in the driving rod accommodating channel (24).

21. The tissue closure device according to claim 16, wherein an engagement opening (25) and a driving opening (26) that are axially spaced are formed on a sidewall of the tube body (1), wherein the driver (6) comprises a fastener (27) and a driving portion (28) which are axially spaced and the driving portion (28) is located in the driving opening (26);
wherein when the fastener (27) is engaged in the engagement opening (25), the driver (6) is in the stabilizer initial position; and
wherein when the fastener (27) is engaged in the driving opening (26), the driver (6) is in the stabilizer triggering position.

22. The tissue closure device according to claim 21, wherein when in the needle triggering position, the needle manipulator (5) blocks the fastener (27) from being detached from the driving opening (26), so as to block the driver (6) from returning from the stabilizer triggering position to the stabilizer initial position.

23. The tissue closure device according to claim 21, wherein the driver (6) comprises a suspension arm (29), the fastener (27) and the driving portion (28) are disposed on the suspension arm (29), and the fastener (27) is located at a proximal end of the suspension arm (29).

24. The tissue closure device according to claim 23, wherein an axially extending guide recess (30) is formed on the needle manipulator (5),
wherein when the needle manipulator (5) is axially moved to the needle initial position, the suspension arm (29) is located on the exterior of the guide recess (30); and
wherein when the needle manipulator (5) is axially moved to the needle triggering position, the suspension arm (29) is slid into the guide recess (30).

25. The tissue closure device according to claim 24, wherein two suspension arms (29) and two guide recesses (30) are provided and are respectively arranged at intervals in a radial direction, and the suspension arms (29) correspond one-to-one to the guide recesses (30), wherein when the needle manipulator (5) is in the needle triggering position, a distal end of the needle manipulator (5) is between the two suspension arms (29); and optionally wherein
the tube body (1) comprises two half tube portions (17) that can be fitted together, and the engagement opening (25) and the driving opening (26) are formed on each of the half tube portions (17).

26. The tissue closure device according to claim 1, wherein at each of the needle outlet channels (2), a wiring notch (31) in communication with the needle outlet channel (2) is formed on the tube body (1),
wherein two of the wiring notches (31) are used to accommodate at least two ends of a suture thread located on the exterior of the tube body (1), respectively; and
wherein when in the needle initial position, a thread-hanging port (40) of each suture needle is at least partially in communication with the wiring notch (31), so that a suture thread can enter the thread-hanging port (40) via the wiring notch (31).

27. The tissue closure device according to claim 26, wherein each of the wiring notches (31) comprises an inlet section (32) and a clamping slit (33), wherein the size of the clamping slit (33) is less than that of the inlet section (32), and when the needle manipulator (5) is in the needle initial position, the clamping slit (33) is configured such that at least two ends of a suture thread clamped within the clamping slit (33) are located in two of the thread-hanging ports (40), respectively.

28. The tissue closure device according to claim 27, wherein the size of the inlet section (32) gradually decreases from the outside to the inside.

29. The tissue closure device according to any one of claims 26 to 28, wherein each of the wiring notches (31) and the needle outlet channel (2) corresponding thereto are arranged in an intersecting manner, at one end of each wiring notch (31), a thread-withdrawing notch (34) that communicates an outlet end of the needle outlet channel (2) with the one end of the wiring notch (31) is formed on the tube body (1), and the thread-withdrawing notch (34) is used to allow a suture thread drawn out when the suture needle (3) extends obliquely from the needle outlet channel (2) to withdraw from the needle outlet channel (2); and optionally wherein:
hollow notches are formed on the tube body (1), and an elastic block (35) is disposed in each hollow notch, wherein the needle outlet channels (2), the wiring notches (31), and the thread-withdrawing notches (34) are formed on the elastic blocks (35).

30. The tissue closure device according to claim 1 or 26, wherein a wiring structure is formed on an outer surface of the tube body (1), and the wiring structure is used to position a suture thread located on the exterior of the tube body (1).

31. The tissue closure device according to claim 30, wherein the wiring structure comprises wiring slots (36) formed on an outer surface of the tube body (1) and extending toward a proximal end of the tube body (1), and the wiring slots (36) are used to accommodate a suture thread located on the exterior of the tube body (1); and optionally wherein:
the wiring slots (36) are in communication with two wiring notches (31) of the tube body (1) that are in communication with two of the needle outlet channels (2).

## Patentansprüche

1. Vorrichtung zum Verschließen von Gewebe, wobei die Vorrichtung umfasst:
einen Röhrenkörper (1), in dem eine Vielzahl von Nadelauslasskanälen (2) ausgebildet sind;
eine Vielzahl von Nahtnadeln (3), die sich in dem Röhrenkörper (1) befinden;
einen Nadelmanipulator (5), der auf dem Röhrenkörper (1) angeordnet ist und zwischen einer Nadelausgangsposition und einer Nadelauslöseposition beweglich ist; und
Stabilisatoren (4) und einen Antrieb (6);
wobei der Nadelmanipulator (5) die Vielzahl von Nahtnadeln (3) so antreiben kann, dass sie sich gleichzeitig schräg aus den entsprechenden Nadelauslasskanälen (2) in eine Punktionsposition erstrecken;
wobei der Nadelmanipulator (5) es ermöglicht, die Vielzahl von Nahtnadeln (3) gleichzeitig zu bewegen, um sie gleichzeitig in die entsprechenden Nadelauslasskanäle (2) zurückzuziehen;
wobei die Stabilisatoren (4) auf dem Röhrenkörper (1) angeordnet und zwischen einer aufgeklappten und einer zusammengeklappten Position beweglich sind;
wobei der Antrieb (6) in dem Röhrenkörper (1) angeordnet und zwischen einer Stabilisatorausgangsposition und einer Stabilisatorauslöseposition beweglich ist;
wobei der Antrieb (6) die Stabilisatoren (4) zur Bewegung veranlassen kann und sich die Stabilisatoren (4) in der Stabilisatorausgangsposition in der zusammengeklappten Position befinden und sich die Stabilisatoren (4) in der Stabilisatorauslöseposition in der ausgeklappten Position befinden;
wobei der Nadelmanipulator (5), wenn er sich in der Nadelausgangsposition befindet, es dem Antrieb (6) ermöglicht, sich zwischen der Stabilisatorauslöseposition und der Stabilisatorausgangsposition zu bewegen; und
**dadurch gekennzeichnet, dass:**
der Nadelmanipulator (5) in der Nadelauslöseposition den Antrieb (6) daran hindert, aus der Stabilisatorauslöseposition in die Stabilisatorausgangsposition zurückzukehren.

2. Vorrichtung zum Verschließen von Gewebe nach Anspruch 1, wobei eine Vielzahl unabhängiger Nahtnadelkanäle (14) in dem Röhrenkörper (1) bereitgestellt ist, jede der Nahtnadeln (3) beweglich in einem jeweiligen Nahtnadelkanal (14) angeordnet ist und jeder der Nahtnadelkanäle (14) einen gekrümmten Kanalabschnitt umfasst, um die Vielzahl von Nahtnadeln (3) so zu führen, dass sich die Nadeln gleichzeitig schräg aus den jeweiligen entsprechenden Nadelauslasskanälen (2) zur Punktionsposition erstrecken können.

3. Vorrichtung zum Verschließen von Gewebe nach Anspruch 2, wobei jeder der Nahtnadelkanäle (14) einen geradlinigen Kanalabschnitt und den gekrümmten Kanalabschnitt umfasst; und gegebenenfalls wobei:
in einer radialen Richtung des Röhrenkörpers (1) der geradlinige Kanalabschnitt auf einer Seite des Röhrenkörpers (1) angeordnet ist, der gekrümmte Kanalabschnitt sich von der einen Seite des Röhrenkörpers (1) zur anderen Seite erstreckt, und ein Ende des gekrümmten Kanalabschnitts als Nadelauslasskanal (2) dient.

4. Vorrichtung zum Verschließen von Gewebe nach Anspruch 2, wobei der Röhrenkörper (1) zwei zusammenfügbare Halbröhrenabschnitte (17) umfasst, an jedem der Halbröhrenabschnitte (17) eine Vielzahl von voneinander unabhängigen Halbnahtnadel-Kanalaussparungen (18) ausgebildet sind und beim Zusammenfügen der beiden Halbröhrenabschnitte (17) zum Röhrenkörper (1) die Vielzahl der Halbnahtnadel-Kanalaussparungen (18) auf einem der Halbröhrenabschnitte (17) und die Vielzahl der Halbnahtnadel-Kanalaussparungen (18) auf dem anderen der Halbröhrenabschnitte (17) jeweils zusammengefügt werden, um die Vielzahl von Nahtnadelkanälen (14) zu bilden; und gegebenenfalls wobei
jede der Nahtnadelkanalaussparungen (18) einen geradlinigen Aussparungsabschnitt (15) umfasst, der sich auf einer Seite in einer radialen Richtung des Röhrenkörpers (1) befindet, und einen gekrümmten Aussparungsabschnitt (16), der sich von der einen Seite zu der anderen Seite erstreckt.

5. Vorrichtung zum Verschließen von Gewebe nach Anspruch 1, wobei die Vorrichtung zum Verschließen von Gewebe eine Nadelspitzen-Schutzstruktur (7) umfasst, die eine zurückgezogene Position und eine Abschirmungsposition aufweist, wobei die Nahtnadeln (3) die Nadelspitzen-Schutzstruktur (7) von der zurückgezogenen Position in die Abschirmungsposition antreiben können, und wobei die Nadelspitzen-Schutzstruktur (7) während des Vorgangs des Antreibens von der zurückgezogenen Position in die Abschirmungsposition und in der Abschirmungsposition die Nadelspitzen der Nahtnadeln (3) abschirmt.

6. Vorrichtung zum Verschließen von Gewebe nach Anspruch 5, wobei die Nadelspitzen-Schutzstruktur (7) eine Schutzoberfläche umfasst, an der die Nadelspitzen der Nahtnadeln (3) anliegen, an der Schutzoberfläche Schutzvorsprünge (37) ausgebildet sind und die Nadelspitzen der Nahtnadeln (3) in der Punktionsposition an den Schutzvorsprüngen (37) angehalten werden.

7. Vorrichtung zum Verschließen von Gewebe nach Anspruch 6, wobei gitterartige Vorsprünge (38) auf der Schutzoberfläche ausgebildet sind und Kanten der gitterartigen Vorsprünge (38) als die Schutzvorsprünge (37) dienen.

8. Vorrichtung zum Verschließen von Gewebe nach Anspruch 6, wobei die Vielzahl von Schutzvorsprüngen (37) in Abständen auf der Schutzoberfläche in einer vom Röhrenkörper (1) wegführenden Richtung angeordnet sind; und gegebenenfalls wobei:
jeder der Schutzvorsprünge (37) gerade verläuft und die Vielzahl von Schutzvorsprüngen (37) wellenförmig oder zickzackförmig angeordnet ist; oder
jeder der Schutzvorsprünge (37) V-förmig verläuft und die Vielzahl von Schutzvorsprüngen (37) wellenförmig oder zickzackförmig angeordnet ist.

9. Vorrichtung zum Verschließen von Gewebe nach Anspruch 6, wobei die Nadelspitzen-Schutzstruktur (7) die Vielzahl von Stabilisatoren (4) und eine Vielzahl von Schutzplatten (42) umfasst, die jeweils die Schutzoberfläche aufweisen, wobei die Schutzplatten (42) eins-zu-eins an den Stabilisatoren (4) bereitgestellt sind, und in der Punktionsposition jede der Nahtnadeln (3) durch einen Nahtfadenbefestigungsabschnitt (39) des jeweiligen entsprechenden Stabilisators (4) hindurch verläuft, um die Schutzplatte (42) aus der zurückgezogenen Position in die Abschirmungsposition zu kippen, wobei in der zurückgezogenen Position die Vielzahl von Schutzplatten (42) innerhalb der jeweiligen entsprechenden Stabilisatoren (4) angeordnet sind und in der Abschirmungsposition die Nadelspitzen der Nahtnadeln (3) an den Schutzvorsprüngen (37) angehalten werden.

10. Vorrichtung zum Verschließen von Gewebe nach Anspruch 9, wobei entweder:
die Schutzplatte (42) ein elastischer Körper ist und unter ihrer eigenen elastischen Kraft aus der Abschirmungsposition in die zurückgezogene Position zurückkehren kann;
die Vielzahl von Stabilisatoren (4) beim Falten die Vielzahl von Schutzplatten (42) drücken kann, so dass die Vielzahl von Schutzplatten (42) aus der Abschirmungsposition in die zurückgezogene Position zurückkehren; oder
die Vielzahl von Schutzplatten (42) jeweils mit den vom Röhrenkörper (1) entfernten Enden der Vielzahl von Stabilisatoren (4) verbunden sind, oder die Vielzahl von Schutzplatten (42) jeweils mit den anderen Enden der Vielzahl von Stabilisatoren (4) nahe dem Röhrenkörper (1) verbunden sind.

11. Vorrichtung zum Verschließen von Gewebe nach Anspruch 6, wobei die Fadenaufhängeöffnungen (40) an den Seitenoberflächen der Vielzahl von Nahtnadeln (3) in der Punktionsposition hin zur Schutzoberfläche ausgerichtet sind.

12. Vorrichtung zum Verschließen von Gewebe nach Anspruch 1, wobei der Nadelmanipulator (5) an einer proximalen Endposition auf dem Röhrenkörper (1) angeordnet ist und sich ein manueller Betätigungsabschnitt (8) des Nadelmanipulators (5) von einem proximalen Endanschluss des Röhrenkörpers (1) erstreckt.

13. Vorrichtung zum Verschließen von Gewebe nach Anspruch 1, wobei der Nadelmanipulator (5) in axialer Richtung des Röhrenkörpers (1) zwischen der Nadelausgangsposition und der Nadelauslöseposition beweglich ist.

14. Vorrichtung zum Verschließen von Gewebe nach Anspruch 13, wobei zwischen dem Nadelmanipulator (5) und dem Röhrenkörper (1) eine Bewegungsführungsstruktur bereitgestellt wird, wobei die Bewegungsführungsstruktur die Nadelausgangsposition und die Nadelauslöseposition des Nadelmanipulators (5) definiert; und gegebenenfalls wobei:
die Bewegungsführungsstruktur einen sich axial erstreckenden Führungsschlitz (9) an einer Außenoberfläche des Nadelmanipulators (5) und einen Führungsvorsprung (10) an einer Innenoberfläche des Röhrenkörpers (1) umfasst, und der Führungsvorsprung (10) in den Führungsschlitz (9) eingepasst ist.

15. Vorrichtung zum Verschließen von Gewebe nach Anspruch 13, wobei eine Vielzahl von radial beabstandeten Nadeleinführöffnungen (11) an einer distalen Endoberfläche des Nadelmanipulators (5) ausgebildet ist, wobei sich die Vielzahl von Nadeleinführöffnungen (11) in Richtung eines proximalen Endes des Nadelmanipulators (5) erstreckt, eine radiale Öffnung (12), die mit der Vielzahl von Nadeleinführöffnungen (11) in Verbindung steht, an dem Nadelmanipulator (5) ausgebildet ist, und ein Verriegelungsstift (13) in die radiale Öffnung (12) eingeführt wird, wobei die Enden der Vielzahl von Nahtnadeln (3) in die jeweiligen Nadeleinführöffnungen (11) eingeführt werden und gleichzeitig mit dem Verriegelungsstift (13) arretiert werden.

16. Vorrichtung zum Verschließen von Gewebe nach Anspruch 1, wobei der Antrieb (6) in axialer Richtung des Röhrenkörpers (1) zwischen der Stabilisatorausgangsposition und der Stabilisatorauslöseposition beweglich ist.

17. Vorrichtung zum Verschließen von Gewebe nach Anspruch 16, wobei eine Vielzahl von radial beabstandeten axialen Kanälen auf dem Antrieb (6) ausgebildet ist und die Vielzahl von Nahtnadeln (3) jeweils die entsprechenden axialen Kanäle durchlaufen.

18. Vorrichtung zum Verschließen von Gewebe nach Anspruch 16, wobei die Vorrichtung zum Verschließen von Gewebe ferner eine Antriebsstange (19) umfasst, die sich in dem Röhrenkörper (1) befindet, wobei ein proximales Ende der Antriebsstange (19) direkt mit dem Antrieb (6) verbunden ist und ein distales Ende der Antriebsstange (19) sich von einem distalen Ende des Röhrenkörpers (1) aus erstreckt, um eine Verbindung mit der Vielzahl von Stabilisatoren (4) herzustellen.

19. Vorrichtung zum Verschließen von Gewebe nach Anspruch 18, wobei eine sich axial erstreckende zentrale Öffnung (20) an einer distalen Endoberfläche des Antriebs (6) ausgebildet ist, ein radialer Kanal (21), der mit der zentralen Öffnung (20) in Verbindung steht, an dem Antrieb (6) ausgebildet ist und ein Verriegelungsschaft (22) in dem radialen Kanal (21) bereitgestellt ist, wobei das proximale Ende der Antriebsstange (19) in die zentrale Öffnung (20) eingeführt und mit dem Verriegelungsschaft (22) arretiert ist.

20. Vorrichtung zum Verschließen von Gewebe nach Anspruch 18, wobei der Röhrenkörper (1) zwei Halbröhrenabschnitte (17) umfasst, die zusammengefügt werden können, eine Halbantriebsstangen-Kanalaussparung (23) an jedem der Halbröhrenabschnitte (17) ausgebildet ist, wenn die beiden Halbröhrenabschnitte (17) zusammengefügt werden, um den Röhrenkörper (1) zu bilden, die Halbantriebsstangen-Kanalaussparung (23) an einem der Halbröhrenabschnitte (17) und die Halbantriebsstangen-Kanalaussparung (23) an dem anderen der Halbröhrenabschnitte (17) so eingepasst werden, dass sie einen Antriebsstangenaufnahmekanal (24) bilden, wobei die Antriebsstange (19) axial beweglich in dem Antriebsstangenaufnahmekanal (24) angeordnet ist.

21. Vorrichtung zum Verschließen von Gewebe nach Anspruch 16, wobei eine Eingriffsöffnung (25) und eine Antriebsöffnung (26), die axial beabstandet sind, an einer Seitenwand des Röhrenkörpers (1) ausgebildet sind, wobei der Antrieb (6) ein Befestigungselement (27) und einen Antriebsabschnitt (28) umfasst, die axial beabstandet sind, und sich der Antriebsabschnitt (28) in der Antriebsöffnung (26) befindet;
wobei, wenn das Befestigungselement (27) in die Eingriffsöffnung (25) eingreift, sich der Antrieb (6) in der Stabilisatorausgangsposition befindet; und
wobei, wenn das Befestigungselement (27) in die Antriebsöffnung (26) eingreift, sich der Antrieb (6) in der Stabilisatorauslöseposition befindet.

22. Vorrichtung zum Verschließen von Gewebe nach Anspruch 21, wobei der Nadelmanipulator (5), wenn er sich in der Nadelauslöseposition befindet, das Lösen des Befestigungselements (27) von der Antriebsöffnung (26) blockiert, um den Antrieb (6) daran zu hindern, von der Stabilisatorauslöseposition in die Stabilisatorausgangsposition zurückzukehren.

23. Vorrichtung zum Verschließen von Gewebe nach Anspruch 21, wobei der Antrieb (6) einen Aufhängungsarm (29) umfasst, das Befestigungselement (27) und der Antriebsabschnitt (28) an dem Aufhängungsarm (29) angeordnet sind und das Befestigungselement (27) an einem proximalen Ende des Aufhängungsarms (29) angeordnet ist.

24. Vorrichtung zum Verschließen von Gewebe nach Anspruch 23, wobei eine sich axial erstreckende Führungsaussparung (30) an dem Nadelmanipulator (5) ausgebildet ist,
wobei, wenn der Nadelmanipulator (5) axial in die Nadelausgangsposition bewegt wird, sich der Aufhängungsarm (29) an der Außenseite der Führungsaussparung (30) befindet; und
wobei, wenn der Nadelmanipulator (5) axial in die Nadelauslöseposition bewegt wird, der Aufhängungsarm (29) in die Führungsaussparung (30) geschoben wird.

25. Vorrichtung zum Verschließen von Gewebe nach Anspruch 24, wobei zwei Aufhängungsarme (29) und zwei Führungsaussparungen (30) bereitgestellt werden, die jeweils in radialer Richtung in Abständen angeordnet sind, und die Aufhängungsarme (29) eins-zu-eins mit den Führungsaussparungen (30) korrespondieren, wobei, wenn sich der Nadelmanipulator (5) in der Nadelauslöseposition befindet, ein distales Ende des Nadelmanipulators (5) zwischen den beiden Aufhängungsarmen (29) angeordnet ist; und gegebenenfalls wobei
der Röhrenkörper (1) zwei Halbröhrenabschnitte (17) umfasst, die zusammengefügt werden können, und die Eingriffsöffnung (25) und die Antriebsöffnung (26) an jedem der Halbröhrenabschnitte (17) ausgebildet sind.

26. Vorrichtung zum Verschließen von Gewebe nach Anspruch 1, wobei an jedem der Nadelauslasskanäle (2) eine mit dem Nadelauslasskanal (2) in Verbindung stehende Verdrahtungskerbe (31) am Röhrenkörper (1) ausgebildet ist,
wobei zwei der Verdrahtungskerben (31) dazu verwendet werden, mindestens zwei Enden eines Nahtfadens aufzunehmen, die sich jeweils an der Außenseite des Röhrenkörpers (1) befinden; und
wobei, wenn sie sich in der Nadelausgangsposition befindet, eine Fadenaufhängeöffnung (40) jeder Nahtnadel mindestens teilweise mit der Verdrahtungskerbe (31) in Verbindung steht, so dass ein Nahtfaden über die Verdrahtungskerbe (31) in die Fadenaufhängeöffnung (40) eintreten kann.

27. Vorrichtung zum Verschließen von Gewebe nach Anspruch 26, wobei jede der Verdrahtungskerben (31) einen Einlassabschnitt (32) und einen Klemmschlitz (33) umfasst, wobei die Größe des Klemmschlitzes (33) geringer ist als die des Einlassabschnitts (32) und der Klemmschlitz (33) so konfiguriert ist, dass sich mindestens zwei Enden eines im Klemmschlitz (33) eingeklemmten Nahtfadens jeweils in zwei der Fadenaufhängeöffnungen (40) befinden, wenn sich der Nadelmanipulator (5) in der Nadelausgangsposition befindet.

28. Vorrichtung zum Verschließen von Gewebe nach Anspruch 27, wobei die Größe des Einlassabschnitts (32) von außen nach innen allmählich abnimmt.

29. Vorrichtung zum Verschließen von Gewebe nach einem der Ansprüche 26 bis 28, wobei jede der Verdrahtungskerben (31) und der damit korrespondierende Nadelauslasskanal (2) überschneidend angeordnet sind, an einem Ende jeder Verdrahtungskerbe (31) eine Fadeneinziehkerbe (34) auf dem Röhrenkörper (1) ausgebildet ist, die ein Auslassende des Nadelauslasskanals (2) mit dem einen Ende der Verdrahtungskerbe (31) verbindet, und die Fadeneinziehkerbe (34) verwendet wird, um zu ermöglichen, dass ein Nahtfaden, der herausgezogen wird, wenn sich die Nahtnadel (3) schräg aus dem Nadelauslasskanal (2) erstreckt, sich aus dem Nadelauslasskanal (2) zurückzieht; und gegebenenfalls wobei:
hohle Kerben auf dem Röhrenkörper (1) ausgebildet sind und ein elastischer Block (35) in jeder hohlen Kerbe angeordnet ist, wobei die Nadelauslasskanäle (2), die Verdrahtungskerben (31) und die Fadeneinziehkerben (34) auf den elastischen Blöcken (35) ausgebildet sind.

30. Vorrichtung zum Verschließen von Gewebe nach Anspruch 1 oder 26, wobei eine Verdrahtungsstruktur auf einer Außenoberfläche des Röhrenkörpers (1) ausgebildet ist und die Verdrahtungsstruktur zum Positionieren eines Nahtfadens verwendet wird, der sich auf der Außenseite des Röhrenkörpers (1) befindet.

31. Vorrichtung zum Verschließen von Gewebe nach Anspruch 30, wobei die Verdrahtungsstruktur Verdrahtungsschlitze (36) umfasst, die auf einer Außenoberfläche des Röhrenkörpers (1) ausgebildet sind und sich in Richtung eines proximalen Endes des Röhrenkörpers (1) erstrecken, und die Verdrahtungsschlitze (36) verwendet werden, um einen Nahtfaden aufzunehmen, der sich auf der Außenseite des Röhrenkörpers (1) befindet; und gegebenenfalls wobei:
die Verdrahtungsschlitze (36) mit zwei Verdrahtungskerben (31) des Röhrenkörpers (1) in Verbindung stehen, die mit zwei der Nadelauslasskanäle (2) in Verbindung stehen.

## Revendications

1. Dispositif de fermeture de tissu, dans lequel le dispositif comprend :
un corps tubulaire (1) dans lequel une pluralité de canaux de sortie d'aiguille (2) sont formés ;
une pluralité d'aiguilles de suture (3) localisées dans le corps tubulaire (1) ;
un manipulateur d'aiguille (5) disposé sur le corps tubulaire (1) et déplaçable entre une position initiale d'aiguille et une position de déclenchement d'aiguille ; et
des stabilisateurs (4) et un entraînement (6) ;
dans lequel le manipulateur d'aiguille (5) peut entraîner la pluralité d'aiguilles de suture (3) à se déplacer simultanément de façon à s'étendre simultanément en oblique à partir de canaux de sortie d'aiguille (2) correspondants respectifs jusqu'à une position de piqûre ;
dans lequel le manipulateur d'aiguille (5) permet à la pluralité d'aiguilles de suture (3) de se déplacer simultanément de façon à se rétracter simultanément dans les canaux de sortie d'aiguille (2) correspondants respectifs ;
dans lequel les stabilisateurs (4) sont disposés sur le corps tubulaire (1) et déplaçables entre une position dépliée et une position pliée ;
dans lequel l'entraînement (6) est disposé dans le corps tubulaire (1) et déplaçable entre une position initiale de stabilisateur et une position de déclenchement de stabilisateur ;
dans lequel l'entraînement (6) peut entraîner les stabilisateurs (4) à se déplacer, et dans la position initiale de stabilisateur, les stabilisateurs (4) sont dans la position pliée, et dans la position de déclenchement de stabilisateur, les stabilisateurs (4) sont dans la position dépliée ;
dans lequel, lorsqu'il est dans la position initiale d'aiguille, le manipulateur d'aiguille (5) permet à l'entraînement (6) de se déplacer entre la position de déclenchement de stabilisateur et la position initiale de stabilisateur ; et
**caractérisé en ce que :**
lorsqu'il est dans la position de déclenchement d'aiguille, le manipulateur d'aiguille (5) empêche l'entraînement (6) de retourner de la position de déclenchement de stabilisateur à la position initiale de stabilisateur.

2. Dispositif de fermeture de tissu selon la revendication 1, dans lequel une pluralité de canaux d'aiguille de suture (14) indépendants sont fournis dans le corps tubulaire (1), chacune des aiguilles de suture (3) est localisée de manière déplaçable dans un canal d'aiguille de suture (14) respectif, et chacun des canaux d'aiguille de suture (14) comprend une section de canal curviligne pour guider la pluralité d'aiguilles de suture (3) de sorte que les aiguilles peuvent s'étendre simultanément en oblique à partir des canaux de sortie d'aiguille (2) correspondants respectifs jusqu'à la position de piqûre.

3. Dispositif de fermeture de tissu selon la revendication 2, dans lequel chacun des canaux d'aiguille de suture (14) comprend une section de canal rectiligne et la section de canal curviligne ; et facultativement dans lequel :
dans une direction radiale du corps tubulaire (1), la section de canal rectiligne est localisée sur un côté du corps tubulaire (1), la section de canal curviligne s'étend à partir du côté précité du corps tubulaire (1) jusqu'à l'autre côté, et une extrémité de la section de canal curviligne sert de canal de sortie d'aiguille (2).

4. Dispositif de fermeture de tissu selon la revendication 2, dans lequel le corps tubulaire (1) comprend deux parties semi-tubulaires (17) qui peuvent s'assembler, une pluralité d'évidements de canal de demi-aiguille de suture (18) mutuellement indépendants sont formés sur chacune des parties semi-tubulaires (17), et lorsque les deux parties semi-tubulaires (17) sont combinées pour former le corps tubulaire (1), la pluralité d'évidements de canal de demi-aiguille de suture (18) sur l'une des parties semi-tubulaires (17) et la pluralité d'évidements de canal de demi-aiguille de suture (18) sur l'autre des parties semi-tubulaires (17) sont assemblées respectivement, de façon à former la pluralité de canaux d'aiguille de suture (14) ; et facultativement dans lequel
chacun des évidements de canal d'aiguille de suture (18) comprend une section évidée rectiligne (15) localisée sur un côté dans une direction radiale du corps tubulaire (1) et une section évidée curviligne (16) s'étendant du côté précité à l'autre côté.

5. Dispositif de fermeture de tissu selon la revendication 1, dans lequel le dispositif de fermeture de tissu comprend une structure de protection de pointe d'aiguille (7) ayant une position rétractée et une position de protection, dans lequel les aiguilles de suture (3) peuvent entraîner la structure de protection de pointe d'aiguille (7) de la position rétractée à la position de protection, et pendant le processus d'entraînement de la position rétractée à la position de protection, et dans la position de protection, la structure de protection de pointe d'aiguille (7) protège les pointes d'aiguille des aiguilles de suture (3).

6. Dispositif de fermeture de tissu selon la revendication 5, dans lequel la structure de protection de pointe d'aiguille (7) comprend une surface de protection pour permettre aux pointes d'aiguille des aiguilles de suture (3) de venir en butée contre celle-ci, des parties saillantes de protection (37) sont formées sur la surface de protection, et dans la position de piqûre, les pointes d'aiguille des aiguilles de suture (3) sont arrêtées au niveau des parties saillantes de protection (37).

7. Dispositif de fermeture de tissu selon la revendication 6, dans lequel des parties saillantes de type grille (38) sont formées sur la surface de protection, et des bords des parties saillantes de type grille (38) servent de parties saillantes de protection (37).

8. Dispositif de fermeture de tissu selon la revendication 6, dans lequel la pluralité de parties saillantes de protection (37) sont agencées à des intervalles sur la surface de protection dans une direction à l'écart du corps tubulaire (1) ; et facultativement dans lequel :
chacune des parties saillantes de protection (37) s'étend linéairement, et la pluralité de parties saillantes de protection (37) sont dans un agencement ondulé ou dans un agencement en zigzag ; ou
chacune des parties saillantes de protection (37) s'étend d'une manière en forme de V, et la pluralité de parties saillantes de protection (37) sont dans un agencement ondulé ou dans un agencement en zigzag.

9. Dispositif de fermeture de tissu selon la revendication 6, dans lequel la structure de protection de pointe d'aiguille (7) comprend la pluralité de stabilisateurs (4) et une pluralité de plaques de protection (42) qui ont chacune la surface de protection, les plaques de protection (42) sont fournies sur les stabilisateurs (4) sur une base univoque, et dans la position de piqûre, chacune des aiguilles de suture (3) passe à travers une partie de fixation de fil de suture (39) du stabilisateur (4) correspondant respectif pour faire basculer la plaque de protection (42) de la position rétractée à la position de protection, dans lequel dans la position rétractée, la pluralité de plaques de protection (42) sont localisées à l'intérieur des stabilisateurs (4) correspondants respectifs, et dans la position de protection, les pointes d'aiguille des aiguilles de suture (3) sont arrêtées au niveau des parties saillantes de protection (37).

10. Dispositif de fermeture de tissu selon la revendication 9, dans lequel soit :
la plaque de protection (42) est un corps élastique et peut retourner de la position de protection à la position rétractée sous sa propre force élastique ;
la pluralité de stabilisateurs (4) peuvent, lorsqu'ils sont pliés, presser la pluralité de plaques de protection (42), de sorte que la pluralité de plaques de protection (42) retournent de la position de protection à la position rétractée ; soit
la pluralité de plaques de protection (42) sont reliées aux extrémités de la pluralité de stabilisateurs (4) à l'écart du corps tubulaire (1) respectivement, soit la pluralité de plaques de protection (42) sont reliées aux autres extrémités de la pluralité de stabilisateurs (4) près du corps tubulaire (1) respectivement.

11. Dispositif de fermeture de tissu selon la revendication 6, dans lequel des orifices de suspension de fil (40) sur des surfaces latérales de la pluralité d'aiguilles de suture (3) sont orientés vers la surface de protection dans la position de piqûre.

12. Dispositif de fermeture de tissu selon la revendication 1, dans lequel le manipulateur d'aiguille (5) est disposé au niveau d'une position d'extrémité proximale sur le corps tubulaire (1), et une partie de fonctionnement manuel (8) du manipulateur d'aiguille (5) s'étend à partir d'un orifice d'extrémité proximal du corps tubulaire (1).

13. Dispositif de fermeture de tissu selon la revendication 1, dans lequel le manipulateur d'aiguille (5) peut être déplacé entre la position initiale d'aiguille et la position de déclenchement d'aiguille dans une direction axiale du corps tubulaire (1).

14. Dispositif de fermeture de tissu selon la revendication 13, dans lequel une structure de guidage de déplacement est fournie entre le manipulateur d'aiguille (5) et le corps tubulaire (1), la structure de guidage de déplacement définissant la position initiale d'aiguille et la position de déclenchement d'aiguille du manipulateur d'aiguille (5) ; et facultativement dans lequel :
la structure de guidage de déplacement comprend une fente de guidage (9) s'étendant axialement sur une surface externe du manipulateur d'aiguille (5) et un bossage de guidage (10) sur une surface interne du corps tubulaire (1), et le bossage de guidage (10) est assemblé dans la fente de guidage (9).

15. Dispositif de fermeture de tissu selon la revendication 13, dans lequel une pluralité de trous d'insertion d'aiguille (11) radialement espacés sont formés sur une surface d'extrémité distale du manipulateur d'aiguille (5), la pluralité de trous d'insertion d'aiguille (11) s'étendent en direction d'une extrémité proximale du manipulateur d'aiguille (5), un trou radial (12) en communication avec la pluralité de trous d'insertion d'aiguille (11) est formé sur le manipulateur d'aiguille (5), et une broche de verrouillage (13) est insérée dans le trou radial (12), dans lequel des extrémités de la pluralité d'aiguilles de suture (3) sont insérées dans les trous d'insertion d'aiguille (11) respectifs et en même temps sont verrouillées en assemblage avec la broche de verrouillage (13).

16. Dispositif de fermeture de tissu selon la revendication 1, dans lequel l'entraînement (6) peut être déplacé entre la position initiale de stabilisateur et la position de déclenchement de stabilisateur dans une direction axiale du corps tubulaire (1).

17. Dispositif de fermeture de tissu selon la revendication 16, dans lequel une pluralité de canaux axiaux radialement espacés sont formés sur l'entraînement (6), et la pluralité d'aiguilles de suture (3) passent à travers les canaux axiaux correspondants respectifs, respectivement.

18. Dispositif de fermeture de tissu selon la revendication 16, dans lequel le dispositif de fermeture de tissu comprend en outre une tige d'entraînement (19) localisée dans le corps tubulaire (1), une extrémité proximale de la tige d'entraînement (19) est directement reliée à l'entraînement (6), et une extrémité distale de la tige d'entraînement (19) s'étend à partir d'une extrémité distale du corps tubulaire (1) pour se relier à la pluralité de stabilisateurs (4).

19. Dispositif de fermeture de tissu selon la revendication 18, dans lequel trou central (20) s'étendant axialement est formé sur une surface d'extrémité distale de l'entraînement (6), un canal radial (21) en communication avec le trou central (20) est formé sur l'entraînement (6), et un arbre de verrouillage (22) est fourni dans le canal radial (21), dans lequel l'extrémité proximale de la tige d'entraînement (19) est insérée dans le trou central (20) et verrouillée en assemblage avec l'arbre de verrouillage (22).

20. Dispositif de fermeture de tissu selon la revendication 18, dans lequel le corps tubulaire (1) comprend deux parties semi-tubulaires (17) qui peuvent s'assembler, un évidement de canal de demi-tige d'entraînement (23) est formé sur chacune des parties semi-tubulaires (17), lorsque les deux parties semi-tubulaires (17) sont combinées pour former le corps tubulaire (1), l'évidement de canal de demi-tige d'entraînement (23) sur l'une des parties semi-tubulaires (17) et l'évidement de canal de demi-tige d'entraînement (23) sur l'autre des parties semi-tubulaires (17) sont assemblés de façon à former un canal de réception de tige d'entraînement (24), dans lequel la tige d'entraînement (19) est disposée de manière déplaçable axialement dans le canal de réception de tige d'entraînement (24).

21. Dispositif de fermeture de tissu selon la revendication 16, dans lequel une ouverture de mise en prise (25) et une ouverture d'entraînement (26) qui sont axialement espacées sont formées sur une paroi latérale du corps tubulaire (1), dans lequel l'entraînement (6) comprend un élément de fixation (27) et une partie d'entraînement (28) qui sont axialement espacés et la partie d'entraînement (28) est localisée dans l'ouverture d'entraînement (26) ;
dans lequel lorsque l'élément de fixation (27) est en prise dans l'ouverture de mise en prise (25), l'entraînement (6) est dans la position initiale de stabilisateur ; et
dans lequel lorsque l'élément de fixation (27) est en prise dans l'ouverture d'entraînement (26), l'entraînement (6) est dans la position de déclenchement de stabilisateur.

22. Dispositif de fermeture de tissu selon la revendication 21, dans lequel lorsqu'il est dans la position de déclenchement d'aiguille, le manipulateur d'aiguille (5) empêche l'élément de fixation (27) de se détacher de l'ouverture d'entraînement (26), de façon à empêcher l'entraînement (6) de retourner de la position de déclenchement de stabilisateur à la position initiale de stabilisateur.

23. Dispositif de fermeture de tissu selon la revendication 21, dans lequel l'entraînement (6) comprend un bras de suspension (29), l'élément de fixation (27) et la partie d'entraînement (28) sont disposés sur le bras de suspension (29), et l'élément de fixation (27) est localisé au niveau d'une extrémité proximale du bras de suspension (29).

24. Dispositif de fermeture de tissu selon la revendication 23, dans lequel un évidement de guidage (30) s'étendant axialement est formé sur le manipulateur d'aiguille (5),
dans lequel lorsque le manipulateur d'aiguille (5) est déplacé axialement à la position initiale d'aiguille, le bras de suspension (29) est localisé sur l'extérieur de l'évidement de guidage (30) ; et
dans lequel lorsque le manipulateur d'aiguille (5) est déplacé axialement à la position de déclenchement d'aiguille, le bras de suspension (29) est coulissé dans l'évidement de guidage (30).

25. Dispositif de fermeture de tissu selon la revendication 24, dans lequel deux bras de suspension (29) et deux évidements de guidage (30) sont fournis et sont respectivement agencés à des intervalles dans une direction radiale, et les bras de suspension (29) correspondent de manière univoque aux évidements de guidage (30), dans lequel lorsque le manipulateur d'aiguille (5) est dans la position de déclenchement d'aiguille, une extrémité distale du manipulateur d'aiguille (5) est entre les deux bras de suspension (29) ; et facultativement dans lequel
le corps tubulaire (1) comprend deux parties semi-tubulaires (17) qui peuvent s'assembler, et l'ouverture de mise en prise (25) et l'ouverture d'entraînement (26) sont formées sur chacune des parties semi-tubulaires (17).

26. Dispositif de fermeture de tissu selon la revendication 1, dans lequel au niveau de chacun des canaux de sortie d'aiguille (2), une encoche de câblage (31) en communication avec le canal de sortie d'aiguille (2) est formée sur le corps tubulaire (1),
dans lequel deux des encoches de câblage (31) sont utilisées pour recevoir au moins deux extrémités d'un fil de suture localisé sur l'extérieur du corps tubulaire (1), respectivement ; et
dans lequel lorsqu'on se trouve dans la position initiale d'aiguille, un orifice de suspension de fil (40) de chaque aiguille de suture est au moins partiellement en communication avec l'encoche de câblage (31), de sorte qu'un fil de suture peut entrer dans l'orifice de suspension de fil (40) par l'intermédiaire de l'encoche de câblage (31).

27. Dispositif de fermeture de tissu selon la revendication 26, dans lequel chacune des encoches de câblage (31) comprend une section d'entrée (32) et une rainure de serrage (33), dans lequel la taille de la rainure de serrage (33) est inférieure à celle de la section d'entrée (32), et lorsque le manipulateur d'aiguille (5) est dans la position initiale d'aiguille, la rainure de serrage (33) est conçue de telle sorte qu'au moins deux extrémités d'un fil de suture serré au sein de la rainure de serrage (33) sont localisées dans deux des orifices de suspension de fil (40), respectivement.

28. Dispositif de fermeture de tissu selon la revendication 27, dans lequel la taille de la section d'entrée (32) diminue progressivement de l'extérieur vers l'intérieur.

29. Dispositif de fermeture de tissu selon l'une quelconque des revendications 26 à 28, dans lequel chacune des encoches de câblage (31) et le canal de sortie d'aiguille (2) correspondant à celles-ci sont agencés d'une manière entrecroisée, au niveau d'une extrémité de chaque encoche de câblage (31), une encoche de retrait de fil (34) qui fait communiquer une extrémité de sortie du canal de sortie d'aiguille (2) avec l'extrémité précitée de l'encoche de câblage (31) est formée sur le corps tubulaire (1), et l'encoche de retrait de fil (34) est utilisée pour permettre à un fil de suture extrait lorsque l'aiguille de suture (3) s'étend en oblique à partir du canal de sortie d'aiguille (2) d'être retiré du canal de sortie d'aiguille (2) ; et facultativement dans lequel :
des encoches creuses sont formées sur le corps tubulaire (1), et un bloc élastique (35) est disposé dans chaque encoche creuse, dans lequel les canaux de sortie d'aiguille (2), les encoches de câblage (31) et les encoches de retrait de fil (34) sont formées sur les blocs élastiques (35).

30. Dispositif de fermeture de tissu selon la revendication 1 ou 26, dans lequel une structure de câblage est formée sur une surface externe du corps tubulaire (1), et la structure de câblage est utilisée pour positionner un fil de suture localisé sur l'extérieur du corps tubulaire (1).

31. Dispositif de fermeture de tissu selon la revendication 30, dans lequel la structure de câblage comprend des fentes de câblage (36) formées sur une surface externe du corps tubulaire (1) et s'étendant en direction d'une extrémité proximale du corps tubulaire (1), et les fentes de câblage (36) sont utilisées pour recevoir un fil de suture localisé sur l'extérieur du corps tubulaire (1) ; et facultativement dans lequel :
les fentes de câblage (36) sont en communication avec deux encoches de câblage (31) du corps tubulaire (1) qui sont en communication avec deux des canaux de sortie d'aiguille (2).
